# EUROPEAN PATENT SPECIFICATION

(11) **EP 3 076 947 B1**
(45) Date of publication and mention of the grant of the patent: **13.06.2018**
(21) Application number: 14837037.2
(22) Date of filing: 04.12.2014
(51) Int. Cl.: A61K 49/04, A61K 9/00, A61K 9/14, A61K 9/16

(54) **CONTRAST MEDIA CONTAINING TASTE MASKING FORMULATIONS**
KONTRASTMITTEL ENTHALTENDE GESCHMACKSABDECKENDE FORMULIERUNGEN
FORMULATIONS DE MASQUAGE DU GOUT CONTENANT UN PRODUIT DE CONTRASTE

(30) Priority: 04.12.2013 US 201361911950 P
(43) Date of publication of application: 12.10.2016
(73) Proprietor: Hovione Scientia Limited, Ringaskiddy, Co. Cork (IE)
(72) Inventor: SMITH, Courtney Rouse, Gainesville, GA 30506 (US); TEMTEM, Marcio, 2975-329 Quinta do Conde (PT); WINTERS, Conrad, 2674-506 Loures (PT)
(74) Representative: Turner, Craig Robert
(86) International application number: PCT/IB2014/003029
(87) International publication number: WO 2015/082998

(56) References cited:
- EP-A1- 1 219 291
- DE-A1- 2 140 177
- FR-A1- 2 848 855
- NL-A- 9 202 136
- US-A- 5 019 370
- US-A1- 2005 180 920
- US-A1- 2006 251 577
- MOHAMED AMIR I ET AL: "Role of PLGA-Nanoparticles Formulation to Improve Drug Radioprotective Activity in Gamma-Irradiated Mice", ACTA FARMACEUTICA BONAERENSE, COLEGIO DE FARMACEUTICOS DE LA PROVINCIA DE BUENOS AIRES, AR, vol. 32, no. 6, 1 August 2013 (2013-08-01) , pages 809-816, XP008176028, ISSN: 0326-2383
- E. PAUWELS ET AL: "An exploratory study of contrast agents for soft tissue visualization by means of high resolution X-ray computed tomography imaging", JOURNAL OF MICROSCOPY, vol. 250, no. 1, 22 April 2013 (2013-04-22), pages 21-31, XP055184457, ISSN: 0022-2720, DOI: 10.1111/jmi.12013
- PAULA CORDEIRO ET AL: "Spray congealing: applications in the Pharmaceutical Industry", CHIMICA OGGI., vol. 31, no. 5, 1 September 2013 (2013-09-01), pages 69-73, XP55183904, IT ISSN: 0392-839X
- AKRE: "Dry Suspension Formulation of Taste Masked Antibiotic Drug for Pediatric Use", JOURNAL OF APPLIED PHARMACEUTICAL SCIENCE, 28 July 2012 (2012-07-28), pages 166-171, XP055183997, DOI: 10.7324/JAPS.2012.2725

## Description

### BACKGROUND OF THE INVENTION

### Field of the Invention

The disclosure provides oral solid particle formulations comprising an iodinated imaging agent and at least one taste masking agent showing improved bioadhesive properties, and are also useful for imaging of the gastrointestinal tract. The invention is defined by the claims. Any subject-matter falling outside the scope of the claims is provided for information purposes only.

### Description of Related Art

Barium sulfate and iodinated contrast media are examples of contrast agents used to enhance computed tomography (CT), magnetic resonance imaging (MR), or conventional radiographic imaging (X-ray) diagnosis. In gastrointestinal radiography applications, the contrasting agents should provide a homogeneous coating of the gastrointestinal mucosa, without interacting with gut secretions and without absorption. Due to the low solubility in water, barium sulfate is typically used to visualize the esophagus, stomach, small and large intestine. But barium sulfate has a generally unpleasant taste that is dependent on the exact makeup of the barium sulfate solution the patient ingests. In addition, the presence of the barium sulfate makes the drink have a mild acidic burning sensation. The consumption of the barium sulfate solution is often considered the worst part of a radiography scan by the patients.

Iodinated contrast agents, such as iohexol and iopamidol, are water-soluble materials mostly used intravenously (for example to improve visualization of blood vessels and lymph nodes). Because of their solubility in water, iodinated contrast agents have not been generally used in the upper gastrointestinal radiography applications.

NL 9202136 relates to preparation for oral use comprising a radiopaque medium selected from the group consisting of sparingly water-soluble iodine-containing aromatic acids and a pharmaceutically acceptable base, the base being present in at least the stoichiometric amount with respect to the acid. The preparation is in solid form, preferably a powder, and should be ingested after having been dissolved in a suitable amount of water

US 2005/180920 relates to a crystalline composition of pharmacologically acceptable non-toxic salt of diatrizoic acid and a low calorie non-sweetened drink mix providing an orally administrable gastrointestinal contrast medium which results in a sufficient and faster rate of contrast in poorly compliant patients undergoing computerized axial tomographic examinations for concentration and subsequent imaging evaluation of an appendix. The non-toxic salt of diatrizoic acid medium may consist of meglumine diatrizoate or sodium diatrizoate and the low calorie non-sweetened drink mix.

US 2006/251577 relates to a contrast agent composition that concentrates in the appendix to enable its direct visualization using computerized axial tomography, a medical diagnostic imaging modality. The composition comprises meglumine diatrizoate, sodium diatrizoate, simethicone, famotidine and aspartame in predetermined amounts that is orally administered to a patient for clinical evaluations of appendicitis. Methods of use include orally administering individual doses of the composition approximately 50 minutes prior to appendix visualization using computerized axial tomography.

### SUMMARY OF THE INVENTION

In a broad aspect, the disclosure provides formulations comprising an iodinated imaging agent and at least one taste masking agent. These formulations are suitable for oral administration and have optimized bioadhesive properties as well as other characteristics such as transit time, cohesion, particle size, surface area, viscosity, and taste / palatability, making them particularly useful for the gastrointestinal radiography applications.

Thus, one aspect of the present invention provides oral formulations comprising an iodinated imaging agent and at least one taste masking agent, wherein the iodinated imaging agent is encapsulated in the taste masking agent into particles, wherein the iodinated imaging agent is a water-soluble organic or polymeric compound that contains one or more of iodine substituents, and wherein the taste masking agent is selected from one or more polymer, surfactant, and sugar.

In another aspect the present invention, provides oral formulations as described herein prepared by a method comprising:
(a) feeding a molten mixture comprising one or more of iodinated imaging agents and one or more of taste masking agents to a nozzle;
(b) atomizing said molten mixture to droplets; and
(c) cooling said droplets to particles.

In another aspect, the present invention provides oral formulations as described herein prepared by a method comprising:
(a) mixing one or more of iodinated agents and one or more of taste making agents in a solvent or mixture of solvents to obtain a solution or a suspension;
(b) feeding and atomizing said solution or suspension into a drying chamber to obtain droplets; and
(c) drying said droplets to particles.

In a further aspect, the present invention provides methods for imaging gastrointestinal tract in a patient, comprising administering to the patient oral formulations of the present invention. The disclosure also provides use of oral formulations of the disclosure for imaging of the gastrointestinal tract.

### BRIEF DESCRIPTION OF DRAWINGS

Encapsulation formulations fit in the claimed scope; dispersion formulations are for reference.
**Figure 1** illustrates spray congealing experimental setup exemplified for Laboratory scale spray dryer in open loop mode.
**Figure 2** illustrates spay drying experimental setup exemplified for a pilot scale spray dryer (with secondary drying step).
**Figure 3** shows Polarized Light Microscopy (PLM) imaging of encapsulation of iopamidol prepared by spray congealing using Precirol ® ATO5.
**Figure 4** shows Scanning Electron Microscopy (SEM) image of encapsulation of iopamidol prepared by spray congealing using Precirol ® ATO5.
**Figure 5** and **Figure 6** show PLM and SEM imaging, respectively, of iopamidol dispersions prepared by spray drying using Eudragit L-100 (poly(methacrylic acid-co-methyl methacrylate)).
**Figure 7** shows SEM imaging of iopamidol dispersions prepared by spray drying using hypromellose acetate succinate (HPMCAS).
**Figure 8** shows SEM imaging of iohexol dispersions prepared by spray drying using Eudragit L-100 (poly(methacrylic acid-co-methyl methacrylate)).
**Figure 9** shows SEM imaging of iohexol dispersions prepared by spray drying using HPMCAS.
**Figure 10** and **Figure 11** show PLM and SEM imaging, respectively, of microencapsulated iopamidol prepared by spray drying using HPMCAS.
**Figure 12** shows SEM imaging of microencapsulated iopamidol prepared by spray drying using Eudragit L-100.
**Figure 13** shows SEM imaging of microencapsulated iohexol prepared by spray drying using HPMCAS.
**Figure 14** shows cohesion values of the materials of the disclosure measured in FT4 powder rheometer at 300 mg Iodine /mL.

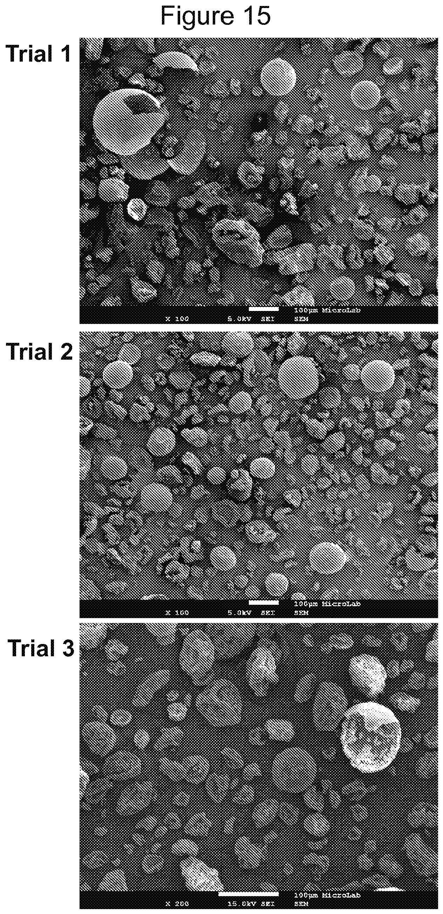

**Figure 15** shows SEM imaging of microencapsulation of iopamidol through spray drying with ultrasonic nozzle using HPMCAS.

### DETAILED DESCRIPTION OF THE INVENTION

In view of the present disclosure, the methods and active materials described herein can be configured by the person of ordinary skill in the art to meet the desired need. In general, the disclosed methods and materials provide improvements in imaging applications, particularly in the gastrointestinal imaging applications. For example, in certain aspect, the oral formulations of the disclosure have optimized characteristics such as transit time, cohesion, bioadhesion, particle size, surface area, viscosity, osmotic pressure, and taste / palatability. In certain embodiments, the formulations of the disclosure also have improved encapsulation efficiency. In other embodiments, the formulations of the disclosure are iso-osmotic or hypo-osmotic.

The oral formulations of the disclosure comprise an iodinated imaging agent and at least one taste masking agent, wherein the iodinated imaging agent and the taste masking agent are formulated into solid particles.

One of skill in the art would recognize that a water soluble iodinated imaging agent could be detected *in vivo* following its administration. In one embodiment, the iodinated imaging agent is an organic or polymeric compound that contains one or more of iodine substituents. Particularly useful iodinated imaging agents include iopamidol (1-*N*,3-*N*-*bis*(1,3-dihydroxypropan-2-yl)-5-[(2*S*)-2-hydroxypropanamido]-2,4,6-triiodobenzene-1,3-dicarboxamide, also known as lopamiro, Isovue, lopamiron, and Niopam), iohexol (1-*N*,3-*N*-bis(2,3-dihydroxypropyl)-5-[*N*-(2,3-dihydroxypropyl)acetamido]-2,4,6-triiodobenzene-1,3-dicarboxamide, also known as Omnipaque), or iodixanol (5-{*N*-[3-(*N*-{3,5-*bis*[(2,3-dihydroxypropyl)carbamoyl]-2,4,6-triiodophenyl}acetamido)-2-hydroxypropyl]acetamido}-1-*N*,3-*N*-*bis*(2,3-dihydroxypropyl)-2,4,6-triiodobenzene-1,3-dicarboxamide, also known as Visipaque). As the person of ordinary skill in the art will appreciate, other iodinated imaging agents may be used. In certain embodiment, the iodinated imaging agent of the disclosure is iopamidol or iohexol. In other embodiments, the iodinated imaging agent of the disclosure is iopamidol. In another embodiment, the iodinated imaging agent of the disclosure is iohexol.

The iodinated imaging agents can be provided in various content, depending, e.g., on the desired end properties of the oral formulations. For example, in certain embodiments, an iodinated imaging agent as described herein is present from about 0.1 to about 90 weight % of the oral formulation. In certain embodiments, a iodinated imaging agent as described herein is present from about 0.1 to about 85 weight %, or about 0.1 to about 80 weight %, or about 0.1 to about 75 weight %, or about 0.1 to about 70 weight %, or about 0.1 to about 60 weight %, or about 0.1 to about 50 weight %, or about 10 to about 90 weight %, or about 10 to about 85 weight %, or about 10 to about 80 weight %, or about 10 to about 75 weight %, or about 10 to about 70 weight %, or about 10 to about 60 weight %, or about 10 to about 50 weight %, or about 0.1 to about 50 weight %,or about 1 to about 50 weight %, or about 5 to about 50 weight %, or about 20 to about 50 weight %, or about 30 to about 50 weight %, or about 40 to about 50 weight %, or about 10 to about 45 weight %, or about 20 to about 45 weight %, or about 10 to about 40 weight %, or about 20 to about 40 weight %, or up to10 weight %, or up to about 20 weight %, or up to about 30 weight %, or up to about 40 weight %, or up to about 50 weight %. In one embodiment, an iodinated imaging agent is present from about 0.5 to about 75 weight % based on the oral formulation. In another embodiment, an iodinated imaging agent is present from about 0.5 to about 90 weight % based on the oral formulation. In another embodiment, an iodinated imaging agent is present up to about 50 weight % based on the oral formulation.

The taste masking agent may be varied depending on the desired formulations. According to the invention, the taste masking agent is selected from one or more polymer, surfactant and sugar. In some embodiments, the oral formulations of the disclosure may further comprise one or more of excipients. Suitable excipients may be selected from the group consisting of surfactants, lipids or waxes, fatty acids, sugars, flavoring agents, and polymers. The excipients may be varied depending on the desired formulations.

The selection of suitable taste masking agents and/or suitable excipients and the quantity to be used for the oral formulation of the present disclosure is within the skill of the person skilled in the art using routine trial and experimentation.

The surfactants, lipids or waxes, and fatty acids suitable for use in the oral formulations of the disclosure include, but are not limited to, hydrogenated castor oil, polyoxyethylene castor oil derivates, mono fatty acid ester of polyoxyethylene sorbitan, polyoxyethylene alkyl ethers, polyoxyethylene alkylaryl ether, polyethylene glycol fatty acid ester, alkylene glycol fatty acid mono ester, sucrose fatty acid ester, sorbitan fatty acid mono ester, polyoxyethyleneglycerol triricinoleate, polyoxyl 35 castor oil (Cremophor®), polyoxyethyleneglycerol oxystearate, mono fatty acids ester of polyoxyethylene sorbitan, PEG-200 monolaurate, PEG-200 dilaurate, PEG-300 dilaurate, PEG-400 dilaurate, PEG-300 distearate, PEG-300 dioleate, propylene glycol monolaurate, D-alpha-tocopheryl polyethylene glycol 1000 succinate, sucrose monostearate, sucrose distearate, sucrose monolaurate, sucrose dilaurate, sorbitan mono laurate, sorbitan monooleate, sorbitan monopalnitate, sorbitan stearate, glyceryl behenate (Compritol®), hydrogenated palm oil, hydrogenated cottonseed oil, lanolin, mono-, di-, and tri alkyl glycerides, polyethylene glycol stearate and polyethylene glycol distearate, macrogolglycerides lauriques (e.g. Gelucire ®), glyceryl palmitostearate (Precirol ®), cetyl alcohol, polyglyceryl diisostearate, glyceryl stearate, palmitic acid, stearyl alcohol, stearic acid, stearic acid, benzoic acid, citric acid, fumaric acid, lactic acid, and maleic acid, lanolin, yellow wax, Carnauba wax, paraffin wax, microcrystalline wax, poloxamers, polysorbates, sodium lauryl sulfate or a combination thereof.

Sugars and flavoring agents useful in the oral formulations of the disclosure include, but are not limited to, aspartame, compressible sugar, dextrates, dextran, dextrin, dextrose, maltodextrinn, calcium carbonate, dibasic calcium phosphate, dextrose, lactose, mannitol, glucose sucrose, maltose, sodium saccharin, sorbitol, tribasic calcium phosphate, calcium sulfate, magnesium carbonate, magnesium oxide, xylitol, and flavors such as banana, grape, vanilla, cherry, eucalyptus oil, menthol, orange, peppermint oil, raspberry, strawberry, and watermelon.

One or more polymers may be included in the oral formulations. Polymers provide bioadhesive strength and may contribute to other properties of the oral formulation. A person skilled in the art will be aware that polymers contribute to the bioadhesive strength, viscosity and application characteristics of the oral formulations. A person skilled in the art will therefore appreciate that the choice of polymer will have an effect on the choice of other ingredients such as other excipients. A skilled artisan will determine the appropriate selection of polymer and the quantity to be used using routine trial and experimentation.

Polymers suitable for use in the oral formulations of the disclosure include, but are not limited to, N-vinyl lactam, copolymer of N-vinyl lactam, cellulose ester, cellulose ether, polyalkylene oxide, polyacrylate, polymethacrylate, poly(methacrylic acid), poly(methyl methacrylate), polyacrylamide, polyvinyl alcohol, vinyl acetate polymer, oligosaccharide, polysaccharide, homopolymer of N-vinyl pyrrolidone, copolymer of N- vinyl pyrrolidone, copolymer of N-vinyl pyrrolidone and vinyl acetate, copolymer of N-vinyl pyrrolidone and vinyl propionate, graft copolymer of polyethylene glycol/polyvinyl caprolactam/polyvinyl acetate (e.g., Soluplus®), polyvinylpyrrolidone, hydroxyalkylcelluloses, hydroxypropylcellulose, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose succinate, hydroxypropylmethylcellulose acetate succinate (e.g. HPMCAS), polyethylene oxide, polypropylene oxide, copolymer of ethylene oxide and propylene oxide, methacrylic acid/ethyl acrylate copolymer, methacrylic acid/methyl methacrylate copolymer (e.g., Eudragit® L 100), butyl methacrylate/2-dimethylaminoethyl methacrylate copolymer, poly(hydroxyalkyl acrylate), crosslinked polyacrylic acid, (e.g. Carbopol ®), poly(hydroxyalkyl methacrylate), copolymer of vinyl acetate and crotonic acid, partially hydrolyzed polyvinyl acetate, carrageenan, galactomannan, or xanthan gum, or a combination thereof.

The oral formulations described herein can be provided in a variety of different particle sizes, depending, e.g., on the methods used for making them and the desired end use. For example, in certain embodiments, an oral formulation as described herein has a particle size within the range of about 1 to about 500 µm, or about 1 to about 400 µm, or about 1 to about 300 µm, or about 1 to about 250 µm, or about 1 to about 200 µm, or about 5 to about 500 µm, or about 5 to about 400 µm, or about 5 to about 300 µm, or about 5 to about 250 µm, or about 10 to about 500 µm, or about 10 to about 250 µm, or about 50 to about 500 µm, or about 50 to about 400 µm, or about 50 to about 300 µm, or about 50 to about 250 µm, or about 100 to about 250 µm, or about 1 to about 100 µm, or about 2 to about 100 µm, or about 5 to about 100 µm, or about 1 to about 50 µm, or about 2 to about 50 µm, or about 5 to about 50 µm, or about 10 to about 50 µm, or about 20 to about 50 µm, or about 30 to about 50 µm, or about 5 to about 40 µm, or about 5 to about 30 µm, or about 5 to about 25 µm, or about 5 to about 20 µm, or about 2 to about 40 µm, or about 2 to about 30 µm, or about 2 to about 25 µm, or about 2 to about 20 µm. In various embodiments, an oral formulation as described herein has a particle size of up to about 5 µm, or up to about 10 µm, or up to about 20 µm, or up to about 40 µm, or up to about 50 µm, or up to about 75 µm, or up to about 100 µm or up to about 150 µm, or up to about 200 µm, or up to about 250 µm, or up to about 300 µm, or up to about 400 µm, or up to about 500 µm in diameter. In particular embodiments, the oral formulation as described herein has a particle size within the range of about 5 to about 50 µm in diameter. In other particular embodiments an oral formulation as described herein has a particle size within the range of about 1 to about 50 µm in diameter. In other embodiments, an oral formulation as described herein has a particle size within the range of about 1 to about 100 µm in diameter. In particular embodiments, an oral formulation as described herein has a particle size within the range of about 50 to about 300 µm. In other particular embodiments an oral formulation as described herein has a particle size within the range of about 50 to about 250 µm. In other embodiments, an oral formulation as described herein has a particle size within the range of about 100 to about 250 µm. The person of ordinary skill in the art can, in view of the methods described herein, provide a desired particle size to an oral formulation.

Similarly, the oral formulation particles described herein can be provided with a variety of different surface areas, depending, e.g., on the methods used for making them and the desired end use. The surface areas are measured using the Brunauer-Emmett-Teller (BET) Surface Area method. In certain embodiments, an oral formulations as described herein has a surface area within the range of from about 0.5 to about 400 m²/g, or about 1 to about 400 m²/g, or about 5 to about 400 m²/g, or about 10 to about 400 m²/g, or about 50 to about 400 m²/g, or about 70 to about 400 m²/g, or about 100 to about 400 m²/g, or about 200 to about 400 m²/g, or about 300 to about 400 m²/g, or about 1 to about 300 m²/g, or about 10 to about 300 m²/g, or about 50 to about 300 m²/g, or about 70 to about 300 m²/g, or about 100 to about 300 m²/g, or about 200 to about 300 m²/g, or about 100 to about 250 m²/g, or about 1 to about 200 m²/g, or about 10 to about 200 m²/g, or about 50 to about 200 m²/g, or about 70 to about 200 m²/g, or about 100 to about 200 m²/g. In other embodiments, an oral formulations as described herein has a surface area within the range of from about 0.5 to about 10 m²/g, or about 1 to about 10 m²/g, or about 2.5 to about 10 m²/g, or about 5 to about 10 m²/g, or about 0.5 to about 9 m²/g, or about 0.5 to about 8 m²/g, or about 0.5 to about 7 m²/g, or about 0.5 to about 5 m²/g, or about 0.75 to about 9 m²/g, or about 0.75 to about 8 m²/g, or about 0.75 to about 7 m²/g, or about 0.75 to about 5 m²/g, or about 1 to about 9 m²/g, or about 1 to about 8 m²/g, or about 1 to about 7 m²/g, or about 1 to about 5 m²/g, or about 1.5 to about 7 m²/g, or about 1.5 to about 5 m²/g, or about 2 to about 7 m²/g, or about 2 to about 5 m²/g, or about 0.5 to about 4 m²/g, or about 0.75 to about 4 m²/g, or about 1 to about 4 m²/g, or about 1.5 to about 4 m²/g, or about 2 to about 4 m²/g, or about 0.5 to about 3 m²/g, or about 0.75 to about 3 m²/g, or about 1 to about 3 m²/g, or about 1.5 to about 3 m²/g, or about 2 to about 3 m²/g, or about 0.5 to about 2.5 m²/g, or about 0.75 to about 2.5 m²/g, or about 1 to about 2.5 m²/g, or about 1.5 to about 2.5 m²/g, or about 2 to about 2.5 m²/g, or up to about 1 m²/g, or up to about 2 m²/g, or up to about 3 m²/g, or up to about 4 m²/g, or up to about 5 m²/g, or up to about 10 m²/g, or up to about 50 m²/g, or up to about 100 m²/g, or up to about 200 m²/g, or up to about 300 m²/g. In one embodiment, an oral formulation as described herein has a surface area of about 0.5 to about 5 m²/g. In another embodiment, an oral formulation as described herein has a surface area of about 1 to about 5 m²/g. In another embodiment, an oral formulation as described herein has a surface area of about 2 to about 4 m²/g. The person of ordinary skill in the art can, in view of the methods described herein, provide a desired surface area to oral formulations.

The oral formulation particles described herein can have various particle to particle cohesions and peak bioadhesive strengths, depending, e.g., on the methods used for making the particles and the desired end use. The particle to particle cohesions and peak bioadhesive strengths are measured using rheological method. For example, in certain embodiments, an oral formulation as described herein has particle to particle cohesion and peak bioadhesive strength within the range of about 0.01 to about 500 kPa, or about 0.01 to about 400 kPa, or about 0.01 to about 300 kPa, or about 0.01 to about 250 kPa, or about 0.1 to about 500 kPa, or about 0.1 to about 250 kPa, or about 10 to about 500 kPa, or about 10 to about 400 kPa, or about 10 to about 300 kPa, or about 10 to about 250 kPa, or about 50 to about 250 kPa. In various embodiments, an oral formulation as described herein has particle to particle cohesion and peak bioadhesive strength of at least about 5 kPa, or at least about 10 kPa, or at least about 50 kPa, or at least about 100 kPa, or at least about 150 kPa, or at least about 200 kPa, or at least about 250 kPa, or at least about 300 kPa, or at least about 400 kPa, or about 500 kPa. In particular embodiments, the oral formulation as described herein has particle to particle cohesion and peak bioadhesive strength within the range of about 10 to about 300 kPa. In other particular embodiments an oral formulation as described herein has particle to particle cohesion and peak bioadhesive strength within the range of about 50 to about 250 kPa. In other embodiments, an oral formulation as described herein has particle to particle cohesion and peak bioadhesive strength within the range of about 100 to about 250 kPa. The person of ordinary skill in the art can, in view of the methods described herein, provide a desired particle to particle cohesion and peak bioadhesive strength to an oral formulation.

In an exemplary embodiment, an iodinated imaging agent is iopamidol or iohexol, and at least one taste masking agent is selected from a surfactant and polymer.

In another exemplary embodiment, an iodinated imaging agent is iopamidol, and at least one taste masking agent is selected from a surfactant and polymer.

In another exemplary embodiment, an iodinated imaging agent is iohexol, and at least one taste masking agent is selected from a surfactant and polymer.

In an exemplary embodiment, an iodinated imaging agent is iopamidol or iohexol, and at least one taste masking agent is selected from hydroxypropylmethylcellulose acetate succinate, poly(methacrylic acid-co-methyl methacrylate, and glyceryl palmitostearate.

In another exemplary embodiment, an iodinated imaging agent is iopamidol, and at least one taste masking agent is selected from hydroxypropylmethylcellulose acetate succinate, poly(methacrylic acid-co-methyl methacrylate, and glyceryl palmitostearate.

In another exemplary embodiment, an iodinated imaging agent is iohexol, and at least one taste masking agent is selected from hydroxypropylmethylcellulose acetate succinate, poly(methacrylic acid-co-methyl methacrylate, and glyceryl palmitostearate. In one aspect, the disclosure provides methods for preparing oral formulations of the disclosure, the method comprising:
(a) feeding a molten mixture comprising one or more of iodinated imaging agents and one or more of taste masking agents to a nozzle;
(b) atomizing said molten mixture to droplets; and
(c) cooling said droplets to particles.

In one embodiment, the method of the disclosure comprises melting one or more of iodinated imaging agents optionally comprising one or more of excipients prior to step (a) to obtain a molten mixture. The molten mixture temperature is determined based on the physicochemical properties of the mixture (e.g., melting temperature). Typical melting temperatures may vary between 20 and 250 °C. Additional mechanical forces, such as shear, may be employed to facilitate the entanglement of the components, as well as to decrease their viscosity.

In one embodiment, the atomization occurs at a nozzle. The feeding of the molten mixture to the nozzle is performed through a heated line to assure consistent temperatures. The nozzle is also heated up to control the molten mixture temperature during atomization. The nozzle types suitable for use in the disclosure include rotary nozzle, pressure nozzles, fluid nozzles, and ultrasonic nozzles. A skilled artisan will determine the appropriate selection of droplet-forming system using routine trial and experimentation.

The controlled cooling can be promoted by a stream gas or liquid. In one embodiment, the stream of gas or liquid is at an initial temperature of between about -40 and about 100 °C. The temperature of the inlet gas determines the cooling rate of the molten droplets, and therefore should be adjusted depending on the system tested. One of skill in the art will recognize that the temperature should be chosen to allow the droplets to solidify in less than about 20 seconds.

In one embodiment, the stream of gas or liquid is co-current or counter-current with respect to the melt spray direction. In another embodiment, the stream of gas or liquid is a stream of gas. The gas suitable for use may be nitrogen, air, carbon dioxide, or mixtures thereof. In another embodiment, the stream of gas or liquid is a stream of gas combined with a stream of liquid. The liquid suitable for use may be liquid carbon dioxide or liquid nitrogen.

In another aspect, the disclosure provides methods for preparing oral formulations of the disclosure, the method comprising:
(a) mixing one or more of iodinated agents and one or more of taste making agents in a solvent or mixture of solvents to obtain a solution or a suspension;
(b) feeding and atomizing said solution or suspension into a drying chamber to obtain droplets; and
(c) drying said droplets to particles.

The drying can be promoted by a stream gas. In one embodiment, the drying is performed at a temperature of between about 20 and about 300 °C. One of skill in the art will recognize that the temperature should be chosen to allow the droplets to dry affording the desired particles.

In one embodiment, the feeding is to a nozzle. The nozzle types suitable for use in the disclosure include, but are not limited to rotary nozzle, pressure nozzles, fluid nozzles, and ultrasonic nozzles. A skilled artisan will determine the appropriate selection of droplet-forming system using routine trial and experimentation.

The controlled drying can be promoted by a stream of gas or liquid. In one embodiment, the stream of gas or liquid is at an initial temperature of between about 25 and about 100 °C. The temperature of the inlet gas determines the drying rate of the molten droplets, and therefore should be adjusted depending on the system tested. One of skill in the art will recognize that the temperature should be chosen to allow the droplets to solidify in less than about 20 seconds. The gas suitable for use may be nitrogen, air, carbon dioxide, or mixtures thereof.

In one embodiment, the stream of gas or liquid is co-current or counter-current with respect to the melt spray direction. In another embodiment, the stream of gas is hot nitrogen.

One aspect of the disclosure provides use of the oral formulations as described herein for imaging of the gastrointestinal tract. One of skill in the art will recognize that the imaging of the gastrointestinal tract includes both, imaging the entire gastrointestinal tract or imaging one or more of individual organs that make up the gastrointestinal tract. For example, one embodiment provides imaging of the entire gastrointestinal tract. Another embodiment provides imaging one or more individual organs of the gastrointestinal tract. In a particular embodiment, the disclosure provides oral formulations for imaging the upper gastrointestinal tract. In one embodiment, the upper gastrointestinal tract includes esophagus, stomach, and duodenum. In another embodiment, esophagus and stomach are imaged. In yet another embodiment, only the stomach is imaged. In another particular embodiment, the disclosure provides oral formulations for imaging the lower gastrointestinal tract. A skilled artisan will determine the appropriate dosage and the time of administration to achieve the desired effect.

### Definitions

The following terms and expressions used have the indicated meanings.

Throughout this specification, unless the context requires otherwise, the word "comprise" and "include" and variations (e.g., "comprises," "comprising," "includes," "including") will be understood to imply the inclusion of a stated component, feature, element, or step or group of components, features, elements or steps but not the exclusion of any other integer or step or group of integers or steps.

As used in the specification and the appended claims, the singular forms "a," "an," and "the" include plural referents unless the context clearly dictates otherwise.

Ranges can be expressed herein as from "about" one particular value, and/or to "about" another particular value.

All percentages, ratios and proportions herein are by weight, unless otherwise specified. A weight percent (weight %, also as wt %) of a component, unless specifically stated to the contrary, is based on the total weight of the formulation or composition in which the component is included (e.g., on the total amount of the formulation). All mol% values are based on the moles of the active compounds.

The term "gastrointestinal tract" as used herein, refers to a series of hollow organs joined in a tube from the mouth to the anus. The hollow organs that make up the gastrointestinal tract are the mouth, esophagus, stomach, small intestine, large intestine (which includes the rectum), and anus. The term "upper gastrointestinal tract" as used herein, refers to the mouth, esophagus, stomach, and, optionally, the duodenum. The term "lower gastrointestinal tract" as used herein, refers to the small intestine, large intestine, and anus.

The term "polymer" as used herein, is synonymous with "copolymer", "heteropolymer" and "alternating copolymer" and means a large molecule (macromolecule) composed of a repeating series of one or more alternating monomeric species. These sub-units are typically connected by covalent chemical bonds.

### EXAMPLES

The methods of the disclosure are illustrated further by the following examples. Encapsulation formulations fit in the claimed scope; dispersion formulations are for reference.

### Example 1: General Methods

### Precirol ® Encapsulation efficiency

To assess morphology and microencapsulation efficiency all the solids produced were analyzed through Scanning Electron Microscopy with Energy Dispersive X-Ray Spectrometry (SEM-EDS). This technique allows the detection of specific atoms on the surface of the powders (0.2-10 µm deep). By calculating the mass percentage of iodine detected on the surface of the materials (from the total mass of iodine, oxygen and carbon detected), and comparing to the values detected in a standard physical mixture of the contrast agent and the excipient, it is possible to estimate the encapsulation efficiency. In the following examples, an encapsulation efficiency of 0 % w/w means the levels of iodine detected are those expected in the physical mixture, and an encapsulation efficiency of 100 % w/w means no iodine is detected. Negative values of encapsulation efficiency mean more contrast agent than that expected in a physical mixture is detected on the surface of the particles. Molecular iodine content of iopamidol and iohexol constitutes 49 %w/w and 46 %w/w of the total molecular weight, respectively.

### Brunauer-Emmett-Teller surface area analysis

By equilibrating each of the powders produced at different nitrogen pressures, the amount of gas that is adsorbed on the surface of the particles can be estimated (Langmuir theory). The total surface area available on the materials is then calculated by approximating the slope and the y-intercept point of the trend line drawn from the different equilibration points.

### Osmotic pressure

The osmotic pressure analysis measures the driving force of a solution to displace water molecules through a membrane from the medium in one side to the other. Considering the membrane is the esophageal epithelium, high osmolality means loss of water from the surrounding tissues to the GI tract.

Samples were diluted to 300 mg Iodine/mL (mg I / mL) in order to perform the osmotic pressure measurement in an osmometer. In the cases where the sample did not produce an aqueous phase to load the sampling cell, dilutions were performed to obtain 15 mg I /mL. The osmolality of the powders produced through micro-encapsulation using spray congealing and spray drying were measured and compared to those of the pure contrast agents.

### Powder rheology

The cohesion of the powder was measured be assessing the rheology of the materials produced. A powder rheometer, from Freeman, model FT4 was used to conducted shear stress tests. The cohesion coefficient for the powders is then estimated by drawing the Mohr circles along the curve obtained from the experimental points of normal versus shear stress. Measurements were performed with powders swelled in water in a concentration of 300 mg l / mL (standard dose used in GI track imaging).

### Melt / Solution / Suspension preparation

Depending on the system three preparations were possible: (1) active pharmaceutical ingredient (API) was suspended in a melt of excipients; (2) API was suspended in a solution where the excipients were previously dissolved; and (3) both API and polymer were dissolved in a common solvent system. Preparation (1) was used to support spray congealing activities while preparations (2) and/or (3) were used to support spray drying.

Preparation (1): Melts were prepared in a heated beaker, with temperature control using a thermic fluid recirculation system. A magnetic stirrer was inserted in the beaker to assist the process and obtain a homogeneous suspension. The following procedure was executed:
(a) the excipient (Precirol ® or Gelucire ®) was weighed and transferred into the beaker;
(b) temperature was increased up to 80°C; and
(c) after the complete melt of the excipient, the contrast agent was suspended in the melt, under agitation.

Preparation (2): Spray drying suspensions were prepared in a glass flask, using a magnetic stirrer to obtain a homogeneous suspension. The procedure was the following:
(a) the process solvent was weighed into the clean, tared, labeled and suitably sized flask.
(b) the polymer /excipient was weighted and transferred into the flask under agitation;
(c) after complete dissolution of the polymer, the contrast agent was weighed and transferred into the flask, under agitation; and
(d) the solution was agitated until obtaining a homogeneous suspension of the contrast agent.

Preparation (3): The spray drying solutions were prepared according to the following procedure:
(a) the process solvent was weighed into the clean, tared, labeled and suitably sized flask.
(b) the polymer was weighted and transferred into the flask under agitation;
(c) after complete dissolution of the polymer, the contrast agent was weighed and transferred into the flask, under agitation; and
(d) the solution was agitated until complete dissolution of the solids.

### Example 2: Spray congealing procedure

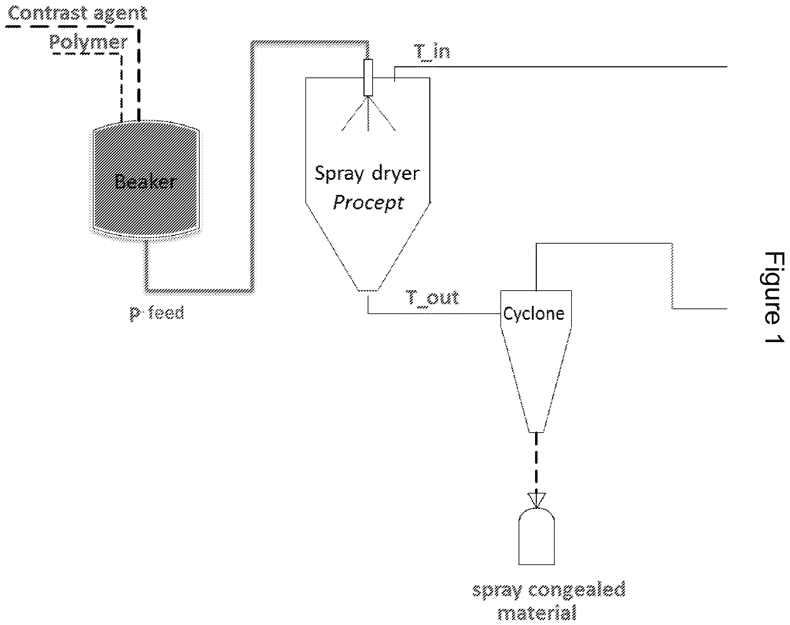

A lab scale spray dryer, equipped with a two-fluid nozzle 1.2 mm orifice assembled, was used to execute the Precirol ® spray congealing tests. The thermal fluid recirculation system was assembled to maintain the temperature of the beaker and heat the feeding line to the tip of the nozzle. The spray congealing unit was operated in open cycle mode, i.e. without recirculation of the congealing gas. A simplified scheme of the spray congealing equipment is shown in Figure 1.

The flow of cooling nitrogen was adjusted to 0.45 m³/min (approximately 30 kg/h). Before initiating the run, the unit was stabilized with gas, adjusting the inlet temperature, T_in, to that estimated for the run.

After stabilization, the melt was fed to the spray dryer to initiate the run. Feeding of the melt was made by pressurizing the beaker. The feed pressure, P_feed, was set to approximately 7 bar.

After atomization in the nozzle, the droplets of melt were cooled and solidified in the drying chamber, by the co-current nitrogen. After leaving the chamber, the stream containing the cooled product enters the cyclone where solid particles are separated from the gas and collected in a glass flask. At the end of the run, the feed was stopped, maintaining the gas flow to perform a controlled shutdown of the unit.

The product collected from the cyclone was weighed and the yield calculated as the mass percentage of the solidified product in relation to the total initial solids.

### Example 3: Spray drying procedure

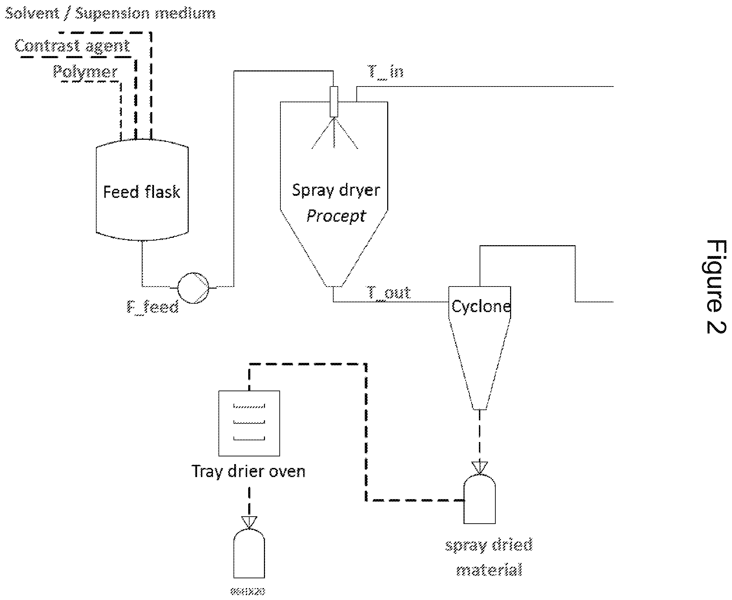

The same lab scale unit, equipped with a two-fluid nozzle and an orifice of 0.8 mm for the spray dried dispersions or an orifice of 1.2 mm for the micro-encapsulation of suspensions, was used to execute the spray drying tests. The spray drying unit was operated in open cycle mode, i.e. without recirculation of the drying gas. A simplified scheme of the spray drying equipment is shown in Figure 2.

The flow of drying nitrogen was adjusted to 0.45 m³/min (approximately 30 kg/h). Before initiating the run, the spray dryer was i) stabilized with drying gas, adjusting the inlet temperature, T_in, to that estimated for the run; and then ii) stabilized with the corresponding solvent, adjusting the feed flow, F_feed, to the correspondent amount of solvent expected to be sprayed in the run. During stabilization with the solvent, T_in was adjusted in order to achieve the target outlet temperature value, T_out, of the run.

After temperatures stabilization, the solution/suspension was fed to the spray dryer to initiate the run. Feeding of the stabilization solvent and of the solution was made through the use of a peristaltic pump, controlling the feed flow rate, F_feed.

After atomization in the nozzle, the droplets of solution/suspension were dried in the drying chamber, by the co-current hot nitrogen. After leaving the drying chamber, the stream containing the dried product enters the cyclone where solid particles are separated from the gas and collected in a glass flask. At the end of the run, the feed was commuted to stabilization solvent in order to rinse the feed line and to perform a controlled shutdown of the unit.

The product collected from the cyclone was weighed and the yield calculated as the mass percentage of the wet product in relation to the total solids in the solution/suspension fed to the spray dryer.

Micro-encapsulation through spray drying using an ultra-sonic nozzle was also performed.

### Example 4: Secondary drying procedure

The materials collected after spray drying were secondarily dried in order to reduce the residual solvents content down to below standard limits (Ethanol: 5000; Methanol: 3000 ppm; Acetone: 5000 ppm). Secondary drying of each batch was conducted during for 24 hours, under vacuum with nitrogen sweep, at 60 °C.

### Example 5: Encapsulation of lopamidol through spray congealing

Table 1 outlines the spray congealing conditions, respective process yield and properties for the lopamidol-containing preparations using Precirol ®.

**Table 1.**

| **Trial** | | **1** | **2** | **3** |
|---|---|---|---|---|
| **Feed solution parameters** | | | | |
| Total solids | g | 40.0 | 40.0 | 40.0 |
| Iopamidol | g | 0 | 8.0 | 16.0 |
| Precirol ® AT05 | g | 40.0 | 32.0 | 24.0 |
| API / polymer ratio | | **Placebo** | **20:80** | **40:60** |

| **Spray congealing parameters** | | | | |
|---|---|---|---|---|
| Nozzle | mm | 1.2 | 1.2 | 1.2 |
| T_feed | °C | 80.0 | 80.0 | 80.0 |
| T_in | °C | 23.6 | 24.8 | 50.0 |
| T_out | °C | 24.6 | - | 38.9 |
| P_feed | bar | Max | Max | Max |
| F_atomiz | mL/min | 9.7 | 15.0 | 25.0 |
| R_atomiz | - | - | - | - |
| F_congealing | m³/min | 0.45 | 0.45 | 0.45 |

| **Process throughput and yield** | | | | |
|---|---|---|---|---|
| Yield | g | 20.6 | 16.4 | - |
| Yield (wet basis) | % | 51.5 | 41.0 | -*^{a)}* |

| **In process results** | | | | |
|---|---|---|---|---|
| Iodine by SEM-EDS (masked material / Physical mixture) | %w/w | - | 2.67/9.8 | 11.38 / 19.6 |
| Encapsulation | % | - | 72.7 | 22.5 |
| Total surface area | m²/g | 0.35 ± 0.02 | 0.53 ± 0.04 | 1.06 ± 0.03 |
| Osmolality *^{c)}* | mOsm/L | - | < LOD | - |

| | | | | |
|---|---|---|---|---|
| *^{a)}* Only an amount of material enough for sampling was considered. API load was tested without *^{b)}* Calculation: ((% *Iodine in the molecule* × % *API)* / *100)* - % *Detected iodine)* / (% *Iodine in the molecule* × % *API* / *100)* *^{c)}* Values measured at a concentration of 15 mg l / mL. | | | | |

Clogging of the nozzle was observed in trial 3. T_in was increased in order to decrease the temperature difference near the nozzle and avoid clogging, but without success. At the end of each trial, a significant amount of material remained in the beaker resulting in the low yield. As known by one skilled in the art, yield could be easily increased by increasing batch size and/or minimizing the amount of material that remains in the beakers and/or adding a more efficient collection mechanism to the apparatus.

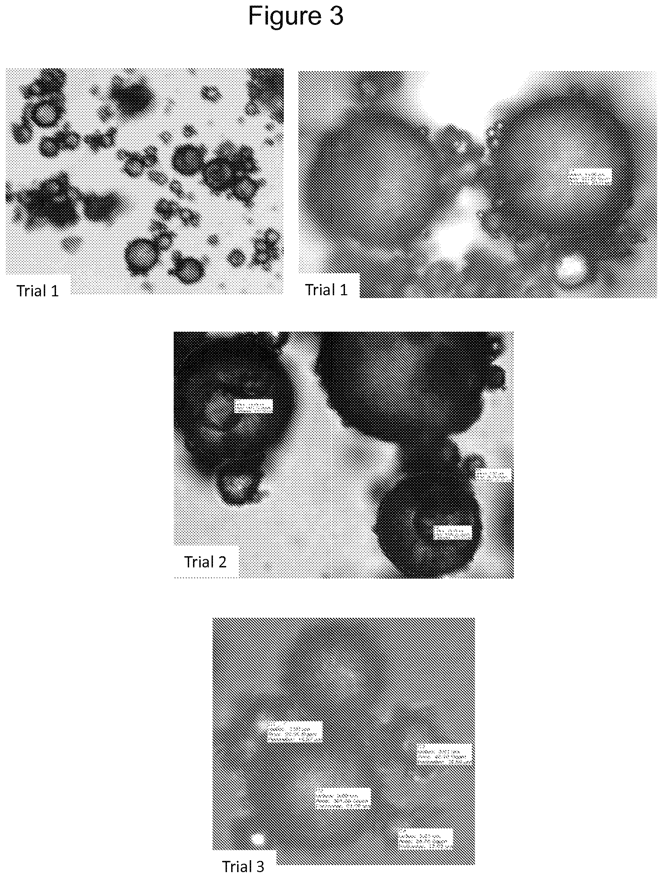

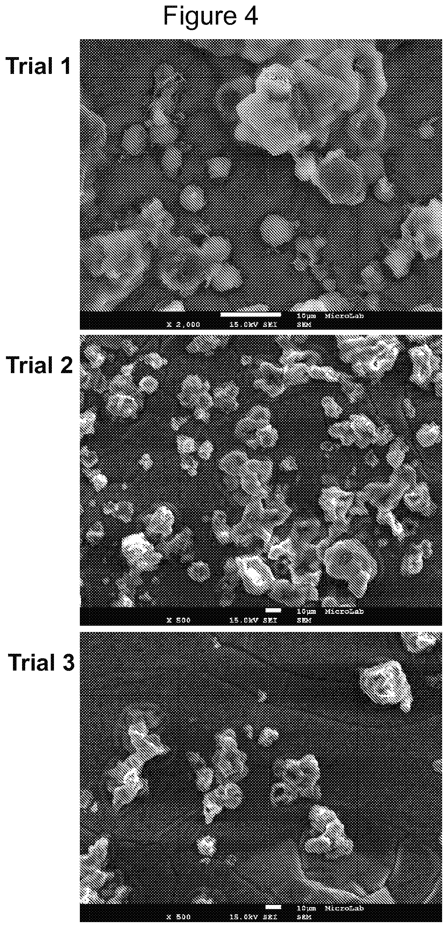

Polarized Light Microscopy (PLM) results are illustrated in Figure 3, which shows that particles are spherical and between 2 µm and 50 µm in size. Scanning Electron Microscopy (SEM) results are shown in Figure 4. The SEM images show irregular particles, with some clusters formed. Without being bound to a particular theory, it is believed that loss of integrity might be due to electron beam and/or sample preparation before analysis and/or a result of the low melting points of the ingredients.

Encapsulation efficiency was 72.7 % and 22.5% for the samples containing 20 and 40 % of contrast agent, respectively. This result suggests that micro-encapsulation of iopamidol through spray congealing delivers good encapsulation efficiency particularly with low iopamidol / excipient ratios.

The materials produced through spray congealing showed low surface area as a result of large particle sizes. See Table 1, Total surface area entry. Moreover, the decrease in the encapsulation efficiency for higher iopamidol load suggests that more iopamidol particles, which have approximately 3 µm in diameter, are not encapsulated, leading to higher surface area.

The sample containing 20 % w/w iopamidol load was also tested for osmotic pressure. The osmotic pressure decreased for the encapsulated material compared to the raw iodinated imaging agents.

### Example 6: Iopamidol dispersions through spray drying

Table 2 outlines the spray drying conditions, respective process yield and properties for the lopamidol-containing preparations using Eudragit® L-100 (poly(methacrylic acid-co-methyl methacrylate).

**Table 2.**

| **Trial** | | **1** | **2** | **3** | **4** |
|---|---|---|---|---|---|
| **Feed solution parameters** | | | | | |
| Total solids | g | 10.0 | 10.0 | 10.0 | 10.0 |
| Iopamidol | g | 0 | 2.0 | 4.0 | 5.0 |
| Eudragit L-100 | g | 10.0 | 8.0 | 6.0 | 5.0 |
| Methanol | g | 190.0 | 190.0 | 190.0 | 190.0 |
| C_feed | %w/w | 5.0 | 5.0 | 5.0 | 5.0 |
| Agent / polymer ratio | | **Placebo** | **20:80** | **40:60** | **50:50** |

| **Spray drying parameters** | | | | | |
|---|---|---|---|---|---|
| Nozzle | mm | 0.8 | 0.8 | 0.8 | 0.8 |
| T_in | °C | 134.0 ± 0.2 | 135.1 ± 0.1 | 135.3 ± 0.3 | 136.8 ± 0.4 |
| T_out | °C | 79.8 ± 0.48 | 80.3 ± 0.8 | 79.4 ± 0.6 | 79.5 ± 0.3 |
| F_feed | g/min | 8.0 | 8.0 | 8.0 | 8.0 |
| F_atomiz | mL/min | 12.0 | 12.0 | 12.0 | 12.0 |
| R_atomiz | - | 1.5 | 1.5 | 1.5 | 1.5 |
| F_drying | m³/min | 0.45 | 0.45 | 0.45 | 0.45 |

| **Process throughput and yield** | | | | | |
|---|---|---|---|---|---|
| Yield | g | 8.1 | 8.2 | 8.0 | 7.9 |

| Yield (wet basis) | % w/w | 81.0 | 82.0 | 80.0 | 79.0 |
|---|---|---|---|---|---|
| **In process results** | | | | | |
| Iodine by SEM-EDS (masked material / Physical mixture) | % w/w | - | 12.45 / 9.8 | 24.60 / 19.6 | 40.90 / 24.5 |
| Encapsulation | % | - | -27.1 | -25.5 | -67.0 |
| Total surface area | m²/g | 3.46 ± 0.07 | 3.31 ± 0.09 | 2.59 ± 0.06 | 2.73 ± 0.08 |
| Osmolality *^{b)}* | mOsm/L | - | - | - | - |

| | | | | | |
|---|---|---|---|---|---|
| *^{a)}* Calculation: ((% *Iodine in the molecule* × % *API)* / *100)* - % *Detected iodine)* / (% *Iodine in the molecule* × % *API* /*100).* Negative values mean that was detected more iodine than expected. *^{b)}* Values measured at a concentration of 15 mg I / mL. | | | | | |

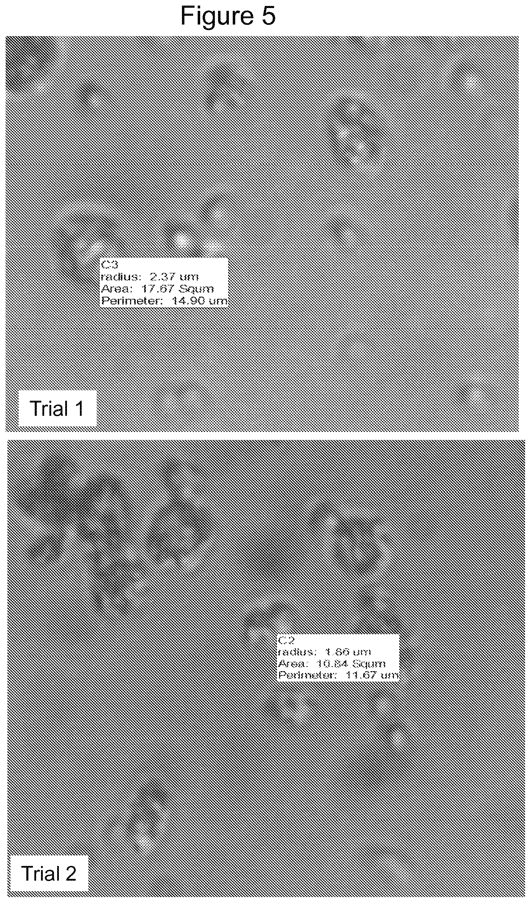

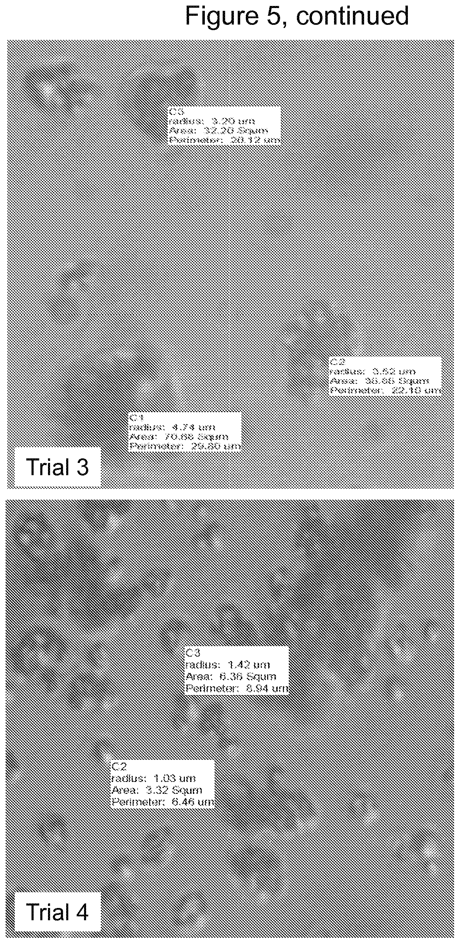

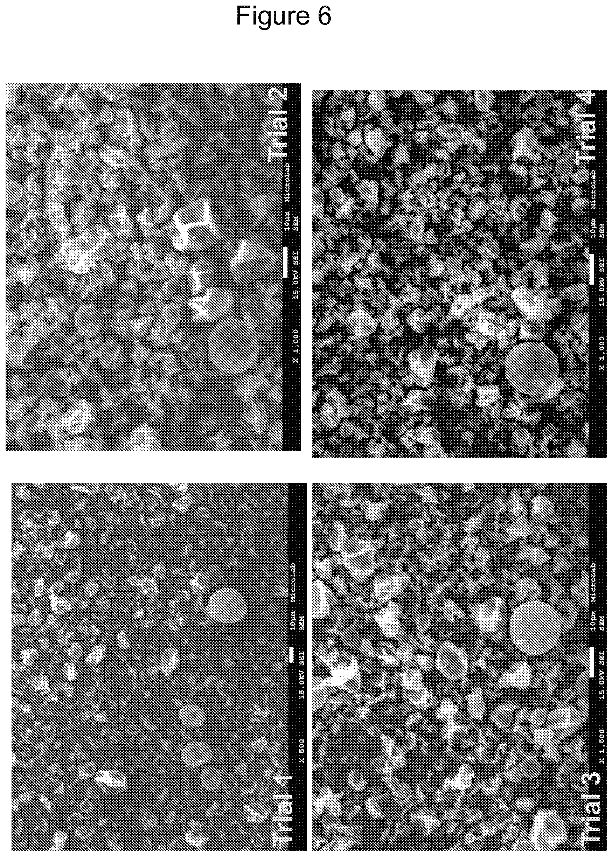

PLM imaging results are illustrated in Figure 5, which shows that particles are between 2 µm and 10 µm in size. SEM imaging results are presented in Figure 6 and show irregular particles. In addition, no degradation was observed.

### Example 7: Iopamidol dispersions through spray drying

Table 3 outlines the spray drying conditions, respective process yield and properties for the lopamidol-containing preparations using HPMCAS (hypromellose acetate succinate, also as hydroxypropylmethylcellulose acetate succinate, also as HPMC-AS).

**Table 3.**

| **Trial** | | **1** | **2** | **3** |
|---|---|---|---|---|
| **Feed solution parameters** | | | | |
| Total solids | g | 10.0 | 10.0 | 10.0 |
| IOPAMIDOL | g | 2.0 | 4.0 | 5.0 |
| HPMCAS | g | 8.0 | 6.0 | 5.0 |
| Methanol | g | 190.0 | 190.0 | 190.0 |
| C_feed | %w/w | 5.0 | 5.0 | 5.0 |
| Agent / polymer ratio | | **20:80** | **40:60** | **50:50** |

| **Spray drying parameters** | | | | |
|---|---|---|---|---|
| Nozzle | mm | 0.8 | 0.8 | 0.8 |
| T_in | °C | 136.0 ± 0.8 | 138.1 ± 0.1 | 137.9 ± 0.2 |
| T_out | °C | 78.6 ± 0.9 | 80.4 ± 0.2 | 79.3 ± 0.6 |
| F_feed | g/min | 8.0 | 8.0 | 8.0 |
| F_atomiz | mL/min | 12.0 | 12.0 | 12.0 |
| R_atomiz | - | 1.5 | 1.5 | 1.5 |
| F_drying | m³/min | 0.45 | 0.45 | 0.45 |

| **Process throughput and yield** | | | | |
|---|---|---|---|---|
| Yield | g | 8.3 | 7.9 | 6.4 |
| Yield (wet basis) | %w/w | 83.0 | 79.0 | 64.0 |

| **In process results** | | | | |
|---|---|---|---|---|
| Iodine by SEM-EDS (masked material / Physical mixture) | %w/w | 20.00 / 9.8 | 33.33 / 19.6 | 37.80/24.5 |
| Encapsulation efficiency | % | -104.1 | -70.1 | -54.3 |
| Total surface area | m²/g | 2.89 ± 0.11 | 2.55 ± 0.08 | 2.18 ± 0.08 |
| Osmolality *^{b)}* | mOsm/L | - | - | - |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* Negative values mean that was detected more iodine than expected. *^{b)}* Values measured at a concentration of 15 mg I / mL. | | | | |

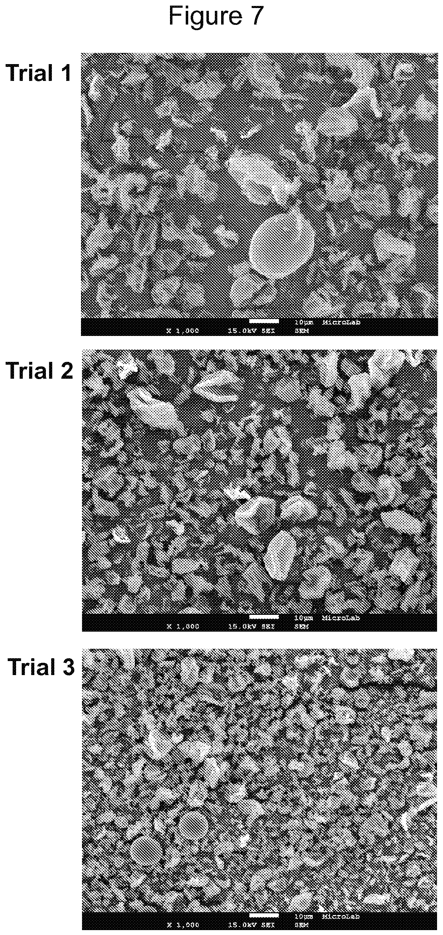

SEM imaging results are presented in Figure 7 and show irregular particles with particle sizes of approximately 10 µm.

### Example 8: Iohexol dispersions through spray drying

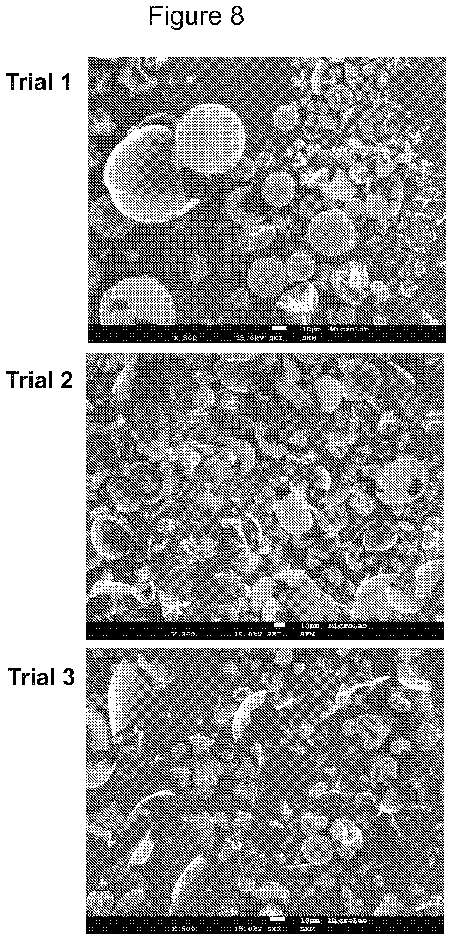

Table 4 outlines the spray drying conditions, respective process yield and properties for the lohexol-containing preparations using Eudragit L-100. Figure 8 shows SEM imaging results.

**Table 4.**

| **Trial** | | **1** | **2** | **3** |
|---|---|---|---|---|
| **Feed solution parameters** | | | | |
| Total solids | g | 20.0 | 20.0 | 20.0 |
| IOHEXOL | g | 4.0 | 8.0 | 10.0 |
| Eudragit L-100 | g | 16.0 | 12.0 | 10.0 |
| Methanol | g | 180.0 | 180.0 | 180.0 |
| C_feed | %w/w | 10.0 | 10.0 | 10.0 |
| Agent / polymer ratio | | **20:80** | **40:60** | **50:50** |

| **Spray drying parameters** | | | | |
|---|---|---|---|---|
| Nozzle | mm | 0.8 | 0.8 | 0.8 |
| T_in | °C | 136.2 ± 0.7 | 140.0 ± 0.0 | 138.1 ± 0.1 |
| T_out | °C | 80.9 ± 0.3 | 80.0 ± 0.7 | 80.0 ± 0.4 |
| F_feed | g/min | 6.5 | 6.5 | 6.5 |
| F_atomiz | mL/min | 6.5 | 6.5 | 6.5 |
| R_atomiz | - | 1.0 | 1.0 | 1.0 |
| F_drying | m³/min | 0.45 | 0.45 | 0.45 |

| **Process throughput and yield** | | | | |
|---|---|---|---|---|
| Yield | g | 8.8 | 10.4 | 17.0 |

| Yield (wet basis) | %w/w | 44.0 | 52.0 | 85.0 |
|---|---|---|---|---|
| **In process results** | | | | |
| Iodine by SEM-EDS (masked material / Physical mixture) | %w/w | 18.79 / 9.3 | 63.39 / 18.5 | 69.48 / 23.2 |
| Encapsulation efficiency | % | -102.6 | -241.8 | -199.8 |
| Total surface area | m²/g | 1.69 ± 0.03 | 1.91 ± 0.07 | 1.96 ± 0.08 |
| Osmolality *^{b)}* | mOsm/L | - | - | - |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* Negative values mean that was detected more iodine than expected. *^{b)}* Values measured at a concentration of 15 mg l / mL. | | | | |

### Example 9: Iohexol dispersions through spray drying

Table 5 outlines the spray drying conditions, respective process yield and properties for the iohexol-containing preparations using HPMCAS.

**Table 5.**

| **Trial** | | **1** | **2** | **3** |
|---|---|---|---|---|
| **Feed solution parameters** | | | | |
| Total solids | g | 24.0 | 20.0 | 20.0 |
| IOHEXOL | g | 8.0 | 8.0 | 10.0 |
| HPMCAS | g | 16.0 | 12.0 | 10.0 |
| Methanol | g | 180.0 | 180.0 | 180.0 |
| C_feed | %w/w | 11.8 | 10.0 | 10.0 |
| Agent / polymer ratio | | **33:67** | **40:60** | **50:50** |

| **Spray drying parameters** | | | | |
|---|---|---|---|---|
| Nozzle | mm | 0.8 | 0.8 | 0.8 |
| T_in | °C | 138.7 ± 0.4 | 139.8 ± 0.2 | 140.0 ± 0.1 |
| T_out | °C | 78.6 ± 0.3 | 79.9 ± 0.1 | 79.6 ± 0.8 |
| F_feed | g/min | 6.5 | 6.5 | 6.5 |
| F_atomiz | mL/min | 12.0 | 12.0 | 12.0 |
| R_atomiz | - | 1.8 | 1.8 | 1.8 |
| F_drying | m³/min | 0.45 | 0.45 | 0.45 |

| **Process throughput and yield** | | | | |
|---|---|---|---|---|
| Yield | g | 20.6 | 16.8 | 15.5 |
| Yield (wet basis) | %w/w | 85.8 | 84.0 | 77.5 |

| **In process results** | | | | |
|---|---|---|---|---|
| Iodine by SEM-EDS (masked material / Physical mixture) | %w/w | 26.27 / 9.3 | 54.37 / 18.5 | 45.35 / 23.2 |
| Encapsulation efficiency | % | -71.7 | -193.2 | -95.6 |
| Total surface area | m²/g | 1.32 ± 0.03 | 1.50 ± 0.04 | 1.70 ± 0.06 |
| Osmolality *^{b)}* | mOsm/L | - | - | - |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* Negative values mean that was detected more iodine than expected. *^{b)}* Values measured at a concentration of 15 mg l / mL. | | | | |

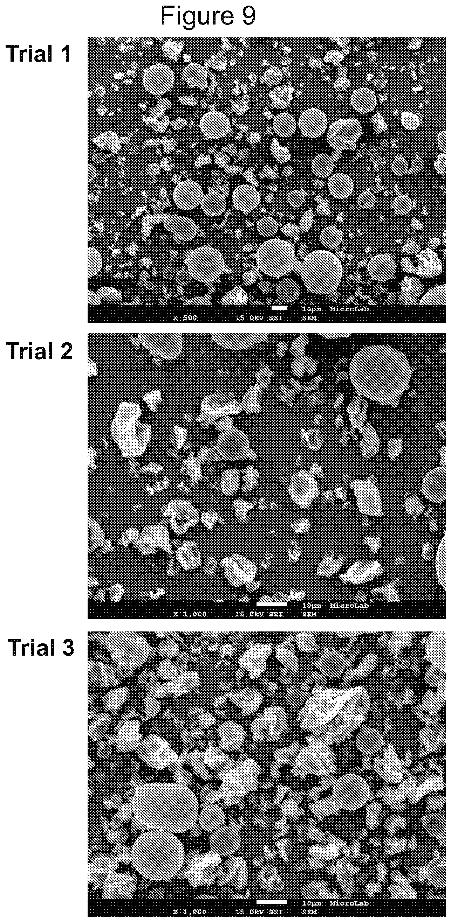

Spherical particles with up to 50 µm in diameter were observed in all trials, and the results are shown in Figure 9.

### Example 10: Encapsulation of lopamidol through spray drying

Table 6 outlines the spray drying conditions, respective process yield and properties for the iopamidol-containing preparations using HPMCAS. The reaction conditions were modified to maintain homogeneously suspended iopamidol during the run.

**Table 6.**

| **Trial** | | **1** | **2** | **3** |
|---|---|---|---|---|
| **Feed solution parameters** | | | | |
| Total solids | g | 20.0 | 20.0 | 20.0 |
| Iopamidol | g | 4.0 | 8.0 | 10.0 |
| HPMCAS | g | 16.0 | 12.0 | 10.0 |
| Acetone | g | 180.0 | 180.0 | 180.0 |
| C_feed | %w/w | 10.0 | 10.0 | 10.0 |
| Agent / polymer ratio | | **20:80** | **40:60** | **50:50** |

| **Spray drying parameters** | | | | |
|---|---|---|---|---|
| Nozzle | mm | 1.2 | 1.2 | 1.2 |
| T_in | °C | 126.0 ± 0.1 | 125.9 ± 0.1 | 135.0 ± 0.1 |
| T_out | °C | 79.0 ± 1.1 | 79.4 ± 0.6 | 82.0 ± 0.3 |
| F_feed | g/min | 6.5 | 6.5 | 6.5 |
| F_atomiz | mL/min | 25.0 | 12.5 | 12.5 |
| R_atomiz | - | 3.9 | 1.9 | 1.9 |
| F_drying | m³/min | 0.45 | 0.45 | 0.45 |

| **Process throughput and yield** | | | | |
|---|---|---|---|---|
| Yield | g | 12.4 | 11.1 | 12.4 |

| Yield (wet basis) | %w/w | 62.0 | 55.0 | 62.0 |
|---|---|---|---|---|
| **In process results** | | | | |
| Iodine by SEM-EDS (masked material / Physical mixture) | %w/w | 3.15 / 9.8 | 12.69 / 19.6 | 18.02 / 24.5 |
| Encapsulation efficiency | % | 67.8 | 41.4 | 26.4 |
| Total surface area | m²/g | 2.11 ± 0.07 | 1.86 ± 0.07 | 0.81 ± 0.05 |
| Osmolality *^{b)}* | mOsm/L | - | 11 | 7 |
| Cohesion *^{c)}* | kPa | 2.93 | 3.42 | 3.96 |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* *^{b)}* Values measured at a concentration of 15 mg l / mL. *^{c)}* Measurements performed with powders swelled in water in a concentration of 300 mg l / mL | | | | |

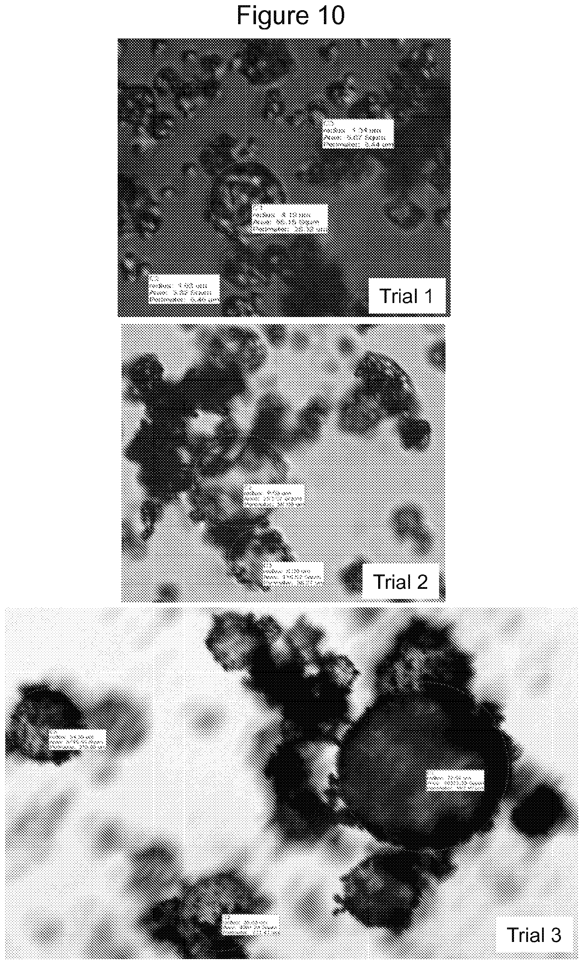

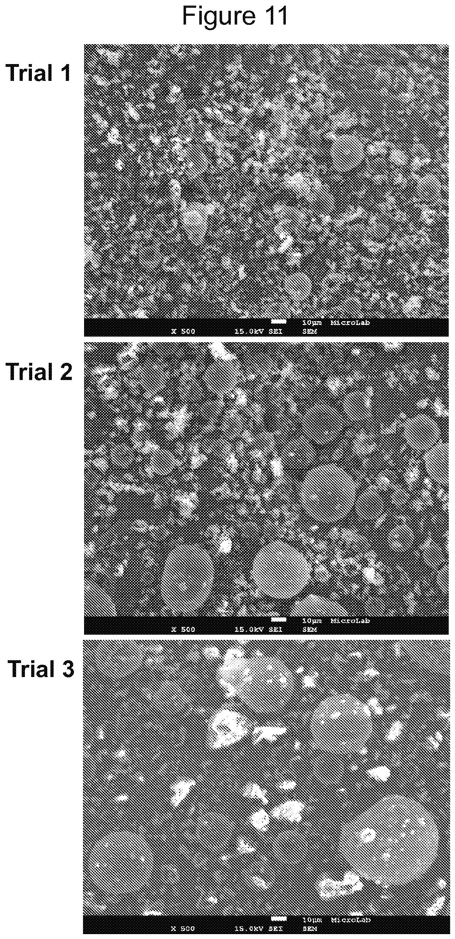

PLM imaging results are illustrated in Figure 10, which shows that particles are between 2 µm and 20 µm in size for trials 1 and 2, and up to 150 µm for trial 3. SEM imaging results are presented in Figure 11 and show irregular particles.

### Example 11: Encapsulation of Iopamidol through spray drying

Table 7 outlines the spray drying conditions, respective process yield and properties for the iopamidol-containing preparations using Eudragit L-100.

**Table 7.**

| **Trial** | | **1** | **2** | **3** |
|---|---|---|---|---|
| **Feed solution parameters** | | | | |
| Total solids | g | 20.0 | 20.0 | 20.0 |
| Iopamidol | g | 4.0 | 8.0 | 10.0 |
| Eudragit L-100 | g | 16.0 | 12.0 | 10.0 |
| Ethanol | | 180.0 | 180.0 | 180.0 |
| C_feed | %w/w | 10.0 | 10.0 | 10.0 |
| Agent / polymer ratio | | **20:80** | **40:60** | **50:50** |

| **Spray drying parameters** | | | | |
|---|---|---|---|---|
| Nozzle | mm | 1.2 | 1.2 | 1.2 |
| T_in | °C | 143.0 ± 0.1 | 130.0 ± 0.1 | 130.0 ± 0.0 |
| T_out | °C | 80.2 ± 1.0 | 81.0 ± 1.5 | 81.0 ± 0.6 |
| F_feed | g/min | 6.5 | 6.5 | 6.5 |
| F_atomiz | mL/min | 7.5 | 7.5 | 7.5 |
| R_atomiz | - | 1.2 | 1.2 | 1.2 |
| F_drying | m³/min | 0.45 | 0.45 | 0.45 |

| **Process throughput and yield** | | | | |
|---|---|---|---|---|
| Yield | g | 18.0 | 12.6 | 12.8 |

| Yield (wet basis) | %w/w | 90.0 | 63.0 | 64.0 |
|---|---|---|---|---|
| **In process results** | | | | |
| Iodine by SEM-EDS (masked material / Physical mixture) | %w/w | 6.32 / 9.8 | 8.42 / 19.6 | 28.57 / 24.5 |
| Encapsulation | % | 35.5 | 57.0 | -16.6 |
| Total surface area | m²/g | 2.61 ± 0.14 | 2.16 ± 0.08 | 2.18 ± 0.07 |
| Osmolality *^{b)}* | mOsm/L | < LOD | < LOD | < LOD |
| Cohesion *^{c)}* | kPa | 1.53 | 2.80 | 3.24 |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* Negative values mean that was detected more iodine than expected. *^{b)}* Values measured at a concentration of 15 mg l / mL. *^{c)}* Measurements performed with powders swelled in water in a concentration of 300 mg l / mL | | | | |

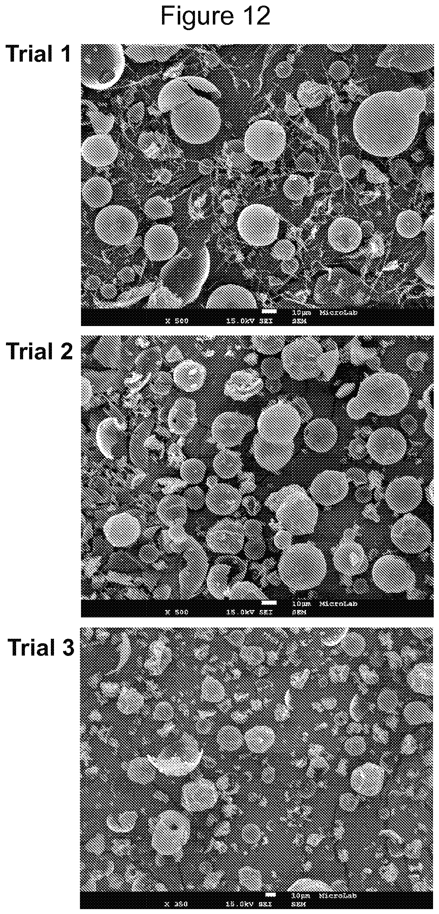

The SEM imaging results are shown in Figure 12.

### Example 12: Encapsulation of Iohexol through spray drying

Table 8 outlines the spray drying conditions, respective process yield and properties for the lohexol-containing preparations using HPMCAS.

**Table 8.**

| **Trial** | | **1** | **2** | **3** |
|---|---|---|---|---|
| **Feed solution parameters** | | | | |
| Total solids | g | 20.0 | 20.0 | 20.0 |
| Iohexol | g | 4.0 | 8.0 | 10.0 |
| HPMCAS | g | 16.0 | 12.0 | 10.0 |
| Acetone | g | 180.0 | 180.0 | 180.0 |
| C_feed | %w/w | 10.0 | 10.0 | 10.0 |
| Agent / polymer ratio | | **20:80** | **40:60** | **50:50** |

| **Spray drying parameters** | | | | |
|---|---|---|---|---|
| Nozzle | mm | 1.2 | 1.2 | 1.2 |
| T_in | °C | 143.3 ± 0.1 | 142.1 ± 0.1 | 143.2 ± 0.0 |
| T_out | °C | 80.7 ± 1.2 | 80.1 ± 0.3 | 79.3 ± 0.2 |
| F_feed | g/min | 6.5 | 6.5 | 6.5 |
| F_atomiz | mL/min | 25.0 | 25.0 | 25.0 |
| R_atomiz | - | 3.8 | 1.2 | 1.2 |
| F_drying | m³/min | 0.45 | 0.45 | 0.45 |

| **Process throughput and yield** | | | | |
|---|---|---|---|---|
| Yield | g | 11.9 | 12.8 | 10.3 |
| Yield (wet basis) | %w/w | 59.0 | 64.0 | 51.5 |

| **In process results** | | | | |
|---|---|---|---|---|
| Iodine by SEM-EDS (masked material / Physical mixture) | %w/w | 0 / 9.3 | 12.59 / 18.5 | 6.18/23.2 |
| Encapsulation efficiency | % | 100.0 | 32.1 | 73.4 |
| Total surface area | m²/g | 1.66 ± 0.06 | 1.21 ± 0.04 | 2.24 ± 0.06 |
| Osmolality *^{b)}* | mOsm/L | - | < LOD | 15 |
| Cohesion *^{c)}* | kPa | 2.18 | - | - |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* *^{b)}* Values measured at a concentration of 15 mg l / mL. *^{c)}* Measurements performed with powders swelled in water in a concentration of 300 mg l / mL | | | | |

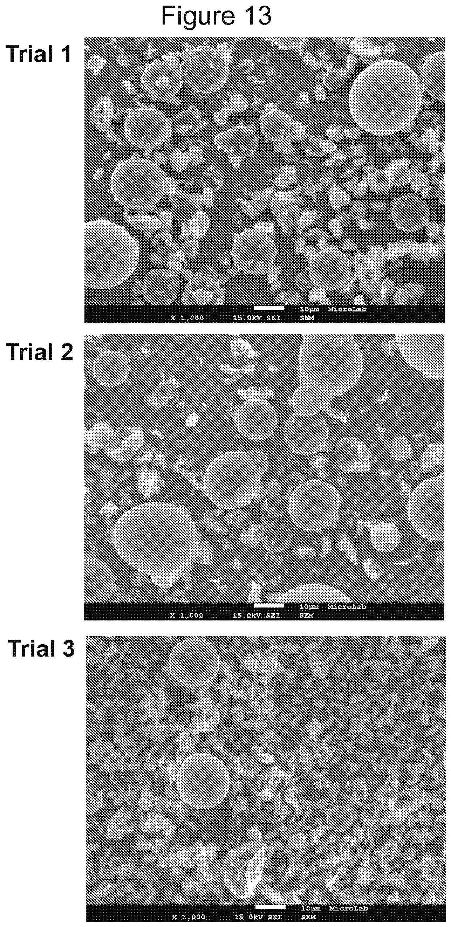

SEM imaging shows particle size of up to 10 µm for trial 1 and trial 2, and smaller for trial 3. Spherical particles with approximately 30 µm in diameter are observed in all trails, decreasing in number for trial 3 (Figure 13).

### Example 13: Analysis of Results of Examples 9-12

High encapsulation efficiencies were obtained for almost all microencapsulates materials obtained by spray drying. Also, all materials produced by microencapsulation through spray drying presented similar surface areas, which were relatively low.

Osmolality values were measured for different formulations to assess the improvement obtained over the pure contrast agents. The osmolality (mOsmol) of iopamidol and iohexol were measured at a concentration of 300 mg I / mL and 15 mg I / mL (Table 9), and compared to that of blood.

**Table 9.**

| | **mOsmol at 300 mg l / mL** | **mOsmol at 15 mg I / mL** |
|---|---|---|
| **Iopamidol (raw)** | 572 | 30 |
| **Iohexol (raw)** | 413 | 36 |
| **blood** | 285-310 | ND |
| **Ex. 9, trial 2 (40 % w/w load)** | ND | 11 |
| **Ex. 10, trial 2 (40 % w/w load)** | ND | < detection limit |
| **Ex. 11, trial 2 (40 % w/w load)** | ND | < detection limit |
| **Ex. 9, trial 3 (50 % w/w load)** | ND | 7 |
| **Ex. 9, trial 3 (50 % w/w load)** | ND | < detection limit |
| **Ex. 11, trial 3 (50 % w/w load)** | ND | 15 |

| | | |
|---|---|---|
| ND: not determined | | |

The microencapsulated materials of the disclosure produced by spray drying methods reduced the osmotic pressure by more than 50 %. Moreover, several materials of the disclosure had osmotic pressure below the detection limit of the equipment.

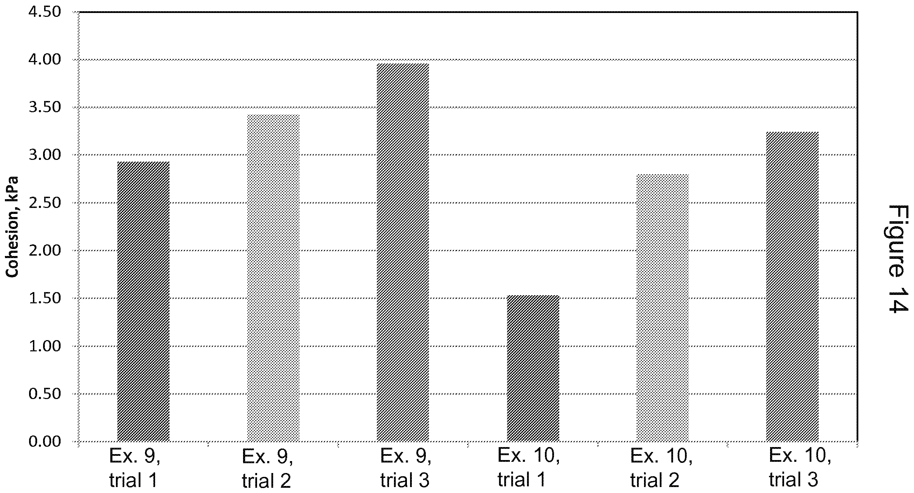

The cohesion of swelled microencapsulates in water 300 mg I /mL (standard dose used in Gl track imaging) was assessed using a powder rheometer (FT4 from Freeman). The results are illustrated in Figure 14. Without being bound to a particular theory, it is believed that the increase in cohesiveness with the API content is correlated with total concentration of API, which was set at 300 mg I /mL.

### Example 14: Encapsulation of lopamidol through spray drying with ultrasonic nozzle

To assess the influence of droplet size in the microencapsulation efficiency the spray drying trials were repeated using an ultrasonic nozzle. Table 10 outlines the spray drying conditions, respective process yield and properties for the iopamidol-containing preparations using HPMCAS. Figure 14 shows SEM imaging results.

**Table 10.**

| **Trail** | | **1** | **2** | **3** |
|---|---|---|---|---|
| **Feed solution parameters** | | | | |
| Total solids | g | 60.0 | 40.0 | 40.0 |
| lopamidol | g | 12.0 | 16.0 | 20.0 |
| HPMCAS | g | 48.0 | 24.0 | 20.0 |
| Acetone | g | 1140.0 | 760.0 | 760.0 |
| C_feed (polymer in | %w/w | 5.0 | 5.0 | 5.0 |
| Agent / polymer ratio | | **20:80** | **40:60** | **50:50** |

| **Spray drying parameters** | | | | |
|---|---|---|---|---|
| Nozzle | (Type) | ultrasonic | ultrasonic | ultrasonic |
| T_in | °C | 100.1 ± 0.1 | 79.0 ± 0.1 | 82.2 ± 0.2 |
| T_out | °C | 74.6 ± 0.2 | 60.1 ± 0.2 | 59.8 ± 0.1 |
| F_feed | g/min | 5.0 | 5.0 | 5.0 |
| Potency_atomiz | W | 5.5 ± 0.5 | 3.0 ± 0.5 | 3.0 ± 0.5 |
| F_drying | m³/min | 0.45 | 0.45 | 0.45 |

| **Process throughput and yield** | | | | |
|---|---|---|---|---|
| Yield | g | 26.6 | 23.8 | 27.6 |

| Yield (wet basis) | %w/w | 44.3 | 59.5 | 69.0 |
|---|---|---|---|---|
| **In process results** | | | | |
| Iodine by SEM-EDS (masked material / physical mixture) | %w/w | 5.23 / 9.8 | 11.34 / 19.6 | 17.93 / 24.5 |
| Encapsulation efficiency | % | 46.6 | 42.1 | 26.8 |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* | | | | |

### Example 15: Encapsulation of lopamidol through spray drying with ultrasonic nozzle

Table 11 outlines the spray drying conditions, respective process yield and properties for the iopamidol-containing preparations using Eudragit L-100. SEM imaging showed particles with up to approximately 100 µm in diameter. Moreover, SEM imaging also showed that most of the API was entrapped inside the shell of the particles.

**Table 11.**

| **Trial** | | **1** | **2** | **3** |
|---|---|---|---|---|
| **Feed solution parameters** | | | | |
| Total solids | G | 40.0 | 40.0 | 40.0 |
| XR01 | G | 8.0 | 16.0 | 20.0 |
| Eudragit L-100 | G | 32.0 | 24.0 | 20.0 |
| Ethanol | G | 760.0 | 760.0 | 760.0 |
| C_feed (polymer in solution) | %w/w | 5.0 | 5.0 | 5.0 |
| Agent / polymer ratio | | **20:80** | **40:60** | **50:50** |

| **Spray drying parameters** | | | | |
|---|---|---|---|---|
| Nozzle | (Type) | ultrasonic | ultrasonic | ultrasonic |
| T_in | °C | 116.0 ± 0.1 | 118.1 ± 0.2 | 117.0 ± 0.2 |
| T_out | °C | 79.9 ± 0.1 | 79.8 ± 0.3 | 79.7 ± 0.1 |
| F_feed | g/min | 5.0 | 5.0 | 5.0 |
| Potency_atomiz | W | 3.0 ± 0.5 | 3.0 ± 0.5 | 2.6 ± 0.5 |
| F_drying | m³/min | 0.45 | 0.45 | 0.45 |

| **Process throughput and yield** | | | | |
|---|---|---|---|---|
| Yield | G | 28.8 | 23.9 | 27.9 |
| Yield (wet basis) | %w/w | 72.0 | 59.8 | 69.8 |

| **In process results** | | | | |
|---|---|---|---|---|
| Iodine by SEM-EDS (masked material / physical mixture) | %w/w | 5.85 / 9.8 | 13.70 / 19.6 | 18.30 / 24.5 |
| Encapsulation efficiency *^{a)}* | % | 40.3 | 30.1 | 25.3 |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* | | | | |

### Example 16: Microencapsulation of lopamidol through spray drying with ultrasonic nozzle

Table 12 outlines the spray drying conditions, respective process yield and properties for the iopamidol-containing preparations using HPMCAS. Figure 14 shows SEM imaging results.

**Table 12.**

| **Trial** | | **1** | **2** | **3** |
|---|---|---|---|---|
| **Feed solution parameters** | | | | |
| Total solids | g | 40.0 | 40.0 | 40.0 |
| XR02 | g | 8.0 | 16.0 | 20.0 |
| HPMCAS | g | 32.0 | 24.0 | 20.0 |
| Acetone | g | 760.0 | 760.0 | 760.0 |
| C_feed (polymer in solution) | %w/w | 5.0 | 5.0 | 5.0 |
| Agent / polymer ratio | | **20:80** | **40:60** | **50:50** |

| **Spray drying parameters** | | | | |
|---|---|---|---|---|
| Nozzle | (Type) | ultrasonic | ultrasonic | ultrasonic |
| T_in | °C | 80.0 ± 0.1 | 80.0 ± 0.1 | 80.0 ± 0.2 |
| T_out | °C | 59.8 ± 0.1 | 59.8 ± 0.1 | 59.8 ± 0.1 |
| F_feed | g/min | 5.0 | 5.0 | 5.0 |
| Potency_atomiz | W | 3.0 ± 0.5 | 3.0 ± 0.5 | 3.0 ± 0.5 |
| F_drying | m³/min | 0.45 | 0.45 | 0.45 |

| **Process throughput and yield** | | | | |
|---|---|---|---|---|
| Yield | g | 26.9 | 26.0 | 27.1 |
| Yield (wet basis) | %w/w | 67.3 | 65.0 | 67.8 |

| **In process results** | | | | |
|---|---|---|---|---|
| Iodine by SEM-EDS (masked material / Physical mixture) | %w/w | 9.47 / 9.3 | 14.89 / 18.5 | 17.41 / 23.2 |
| Encapsulation efficiency *^{a)}* | % | -2.1 | 19.7 | 24.9 |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* Negative values mean that was detected more iodine than expected. | | | | |

### Example 17: Encapsulation of Iopamidol through spray drying with ultrasonic nozzle

The solvent system used to solubilize Eudragit L-100 while maintaining loexol (XR02) in suspension was acetone with 3.0 % w/w of deionized water. Table 13 outlines the spray drying conditions, respective process yield and properties for the iohexol-containing preparations using Eudragit L-100.

**Table 13.**

| **Trial** | | **1** | **2** | **3** |
|---|---|---|---|---|
| **Feed solution parameters** | | | | |
| Total solids | g | 40.0 | 40.0 | 40.0 |
| XR02 | g | 8.0 | 16.0 | 20.0 |
| Eudragit L-100 | g | 32.0 | 24.0 | 20.0 |
| Acetone (with 3 %w/w water) | g | 760.0 | 760.0 | 760.0 |
| C_feed (polymer in solution) | %w/w | 5.0 | 5.0 | 5.0 |
| Agent / polymer ratio | | **20:80** | **40:60** | **50:50** |

| **Spray drying parameters** | | | | |
|---|---|---|---|---|
| Nozzle | (Type) | ultrasonic | ultrasonic | ultrasonic |
| T_in | °C | 80.0 ± 0.1 | 80.1 ± 0.1 | 80.0 ± 0.1 |
| T_out | °C | 60.0 ± 0.1 | 59.9 ± 0.1 | 59.9 ± 0.1 |
| F_feed | g/min | 5.0 | 5.0 | 5.0 |
| Potency_atomiz | W | 3.0 ± 0.5 | 3.0 ± 0.5 | 3.0 ± 0.5 |
| F_drying | m³/min | 0.45 | 0.45 | 0.45 |

| **Process throughput and yield** | | | | |
|---|---|---|---|---|
| Yield | G | 31.9 | 32.7 | 30.5 |
| Yield (wet basis) | %w/w | 79.8 | 81.8 | 76.3 |

| **In process results** | | | | |
|---|---|---|---|---|
| Iodine by SEM-EDS (masked material / Physical | %w/w | 6.06 / 9.3 | 15.5 / 18.5 | 19.05 / 23.2 |
| Encapsulation efficiency *^{a)}* | % | 34.6 | 16.3 | 17.8 |

| | | | | |
|---|---|---|---|---|
| *^{a)}* Calculation: ((% *Iodine in the molecule* × % *API)* / *100)* - % *Detected iodine)* / (% *Iodine in the molecule* × % *API* / *100).* | | | | |

## Claims

1. An oral formulation comprising an iodinated imaging agent and at least one taste masking agent, wherein the iodinated imaging agent is encapsulated in the taste masking agent into particles, wherein the iodinated imaging agent is a water-soluble organic or polymeric compound that contains one or more of iodine substituents, and wherein the taste masking agent is selected from one or more polymer, surfactant, and sugar.

2. An oral formulation of claim 1, wherein the imaging agent is iopamidol, iohexol, or iodixanol.

3. An oral formulation of claim 1 or 2, wherein the imaging agent is present up to 90% by weight.

4. An oral formulation of claim 1, further comprising any one or more of the following feature:
(i) wherein one or more of taste masking agent is selected from hydroxypropylmethylcellulose acetate succinate, poly(methacrylic acid-co-methyl methacrylate), and glyceryl palmitostearate;.
(ii) wherein the taste masking agent is hydroxypropylmethylcellulose acetate succinate;
(iii) wherein the taste masking agent is poly(methacrylic acid-co-methyl methacrylate).

5. An oral formulation of any one of claims 1-4, further comprising one or more of excipients, wherein the excipient is selected from the group consisting of polymers, surfactants, lipids or waxes, fatty acids, sugars, and flavoring agents.

6. An oral formulation of any one of claims 1-5, wherein the particles have at least one of the following features:
(i) a particle size in the range of between 1 and 500 µm diameter;
(ii) a surface area between 0.5 and 100 m²/g as measured using the Brunauer-Emmett-Teller (BET) Surface Area method;
(iii) a particle to particle cohesion and peak bioadhesive strength of up to 500 kPa as measured using a powder rheometer.

7. An oral formulation of any of claim 1-6, wherein the formulation is iso-osmotic or hypo-osmotic.

8. A method for preparing a formulation of any one of claims 1-7, the method comprising:
(a) feeding a molten mixture comprising one or more of iodinated imaging agents and one or more of taste masking agents to a nozzle;
(b) atomizing said molten mixture to droplets; and
(c) cooling said droplets to particles.

9. The method of claim 8, further comprising melting one or more of iodinated imaging agents optionally comprising one or more of excipients prior to step (a), to obtain the molten mixture; wherein optionally the melting is done at a temperature of between 20 to 250 °C.

10. The method of claim 8 or 9, wherein cooling is promoted by a stream of gas or liquid, optionally wherein the stream of gas or liquid is any one of the following:
(i) said stream of gas or liquid is at an initial temperature of between-40 and 100 °C;
(ii) said stream of gas or liquid is co-current or counter-current with respect to the melt spray direction;
(iii) said stream of gas or liquid is a stream of gas comprising nitrogen, air, carbon dioxide, or mixtures thereof;
(iv) said stream of gas or liquid is a stream of gas combined with a stream of liquid comprising liquid carbon dioxide or liquid nitrogen.

11. A method for preparing a formulation of any one of claims 1-7, the method comprising:
(a) mixing one or more of iodinated agents and one or more of taste making agents in a solvent or mixture of solvents to obtain a solution or a suspension;
(b) feeding and atomizing said solution or suspension into a drying chamber to obtain droplets; optionally the feeding is to a nozzle and
(c) drying said droplets to particles.

12. The method of claim 11, wherein drying is promoted by a stream of gas or liquid, optionally, the drying is done at a temperature of between 20 and 300 °C.

13. The method of any one of claims 8, 9, 11 or 12, wherein the nozzle is of rotary, pressure, fluid, or ultrasonic configuration.

14. The method of claim 12, further comprising any one or more of the following feature:
(i) wherein the stream of gas or liquid is co-current with respect to the melt spray direction;
(ii) wherein the stream of gas comprises nitrogen, air, carbon dioxide, or mixtures thereof;
(iii) wherein the stream of gas is hot nitrogen.

15. A method for imaging gastrointestinal tract in a patient, comprising administering to the patient an oral formulation according to any one of claims 1-7, optionally the gastrointestinal tract is upper gastrointestinal tract.

## Patentansprüche

1. Orale Formulierung, ein jodiertes Kontrastmittel und zumindest ein Geschmacksmaskierungsmittel umfassend, wobei das jodierte Kontrastmittel als Partikel im Geschmacksmaskierungsmittel eingekapselt ist, wobei das jodierte Kontrastmittel eine wasserlösliche organische oder polymerische Verbindung ist, die einen oder mehrere Jodsubstituenten beinhaltet, und wobei das Geschmacksmaskierungsmittel aus einem oder mehreren Polymeren, Tensiden und Zuckern ausgewählt wird.

2. Orale Formulierung nach Anspruch 1, wobei das Kontrastmittel Iopamidol, Iohexol oder Iodixanol ist.

3. Orale Formulierung nach Anspruch 1 oder 2, wobei das Kontrastmittel bis zu 90 % des Gewichts ausmacht.

4. Orale Formulierung nach Anspruch 1, ferner umfassend jedwedes oder mehrere der folgenden Merkmale:
(i) wobei eines oder mehrere der Geschmacksmaskierungsmittel aus Hydroxypropylmethylcelluloseazetatsuccinat, Poly(methacrylsäure-Komethylmethacrylat) und Glycerinpalmitostearat ausgewählt werden;.
(ii) wobei das Geschmacksmaskierungsmittel Hydroxypropylmethylcelluloseazetatsuccinat ist;
(iii) wobei das Geschmacksmaskierungsmittel Poly(methacrylsäure-Komethylmethacrylat) ist.

5. Orale Formulierung nach einem der Ansprüche 1-4, ferner umfassend einen oder mehrere Hilfsstoffe, wobei der Hilfsstoff aus der Gruppe ausgewählt wird, die aus Polymeren, Tensiden, Lipiden oder Wachsen, Fettsäuren, Zuckern und Geschmacksstoffen besteht.

6. Orale Formulierung nach einem der Ansprüche 1-5, wobei die Partikel zumindest eines der folgenden Merkmale aufweisen:
(i) Partikelgröße im Bereich zwischen 1 und 500 µm Durchmesser;
(ii) eine Oberfläche zwischen 0,5 und 100 m²/g bei Messung unter Verwendung der Brunauer-Emmet-Teller (BET)-Oberflächenmethode;
(iii) eine Partikel-zu-Partikel-Kohäsion und eine Spitzenbioadhäsionsstärke von bis zu 500 kPa bei Messung mit einem Pulverrheometer.

7. Orale Formulierung nach einem der Ansprüche 1-6, wobei die Formulierung isoosmotisch oder hypoosmotisch ist.

8. Verfahren zur Präparation einer Formulierung nach einem der Ansprüche 1-7, das Verfahren umfassend:
(a) Beschicken einer Düse mit einer geschmolzenen Mischung, die ein oder mehrere jodierte Kontrastmittel und ein oder mehrere Geschmacksmaskierungsmittel umfasst;
(b) Zerstäuben dieser geschmolzenen Mischung zu Tröpfchen; und
(c) Abkühlen dieser Tröpfchen zu Partikeln.

9. Verfahren nach Anspruch 8, ferner umfassend ein Schmelzen eines oder mehrerer der jodierten Kontrastmittel, die optional einen oder mehrere Hilfsstoffe umfassen, vor Schritt (a), um eine geschmolzene Mischung zu erhalten; wobei das Schmelzen optional bei einer Temperatur zwischen 20 und 250 °C durchgeführt wird.

10. Verfahren nach Anspruch 8 oder 9, wobei ein Abkühlen durch einen Strom von Gas oder Flüssigkeit gefördert wird, wobei optional der Strom von Gas oder Flüssigkeit jedwedes der Folgenden ist:
(i) dieser Strom von Gas oder Flüssigkeit hat eine Anfangstemperatur von zwischen -40 und 100 °C;
(ii) dieser Strom von Gas oder Flüssigkeit ist gleichlaufend oder gegenläufig zur Richtung der gesprühten Schmelze;
(iii) dieser Strom von Gas oder Flüssigkeit ist ein Gasstrom, der Stickstoff, Luft, Kohlendioxid oder Mischungen davon umfasst;
(iv) dieser Strom von Gas oder Flüssigkeit ist ein Gasstrom, der mit einem Flüssigkeitsstrom kombiniert ist, der flüssiges Kohlendioxid oder flüssigen Stickstoff umfasst.

11. Verfahren zur Präparation einer Formulierung eines der Ansprüche 1-7, das Verfahren umfassend:
(a) Mischen eines oder mehrerer der jodierten Mittel und eines oder mehrerer der Geschmacksmaskierungsmittel in ein Lösungsmittel oder eine Lösungsmittelmischung, um eine Lösung oder eine Suspension zu erhalten;
(b) Beschicken und Zerstäuben dieser Lösung oder Suspension in eine Trockenkammer, um Tröpfchen zu erhalten; optional ist das Beschicken an eine Düse; und
(c) Trocknen dieser Tröpfchen zu Partikeln.

12. Verfahren nach Anspruch 11, wobei ein Trocknen durch einen Strom von Gas oder Flüssigkeit gefördert wird, optional wird das Trocknen bei einer Temperatur zwischen 20 und 300 °C durchgeführt.

13. Verfahren nach einem der Ansprüche 8, 9, 11 oder 12, wobei die Düse eine Rotations-, Druck-, Fluid- oder Ultraschallkonfiguration aufweisen kann.

14. Verfahren nach Anspruch 12, ferner umfassend eines oder mehrere der folgenden Merkmale:
(i) wobei der Strom von Gas oder Flüssigkeit gleichlaufend zur Richtung der gesprühten Schmelze ist;
(ii) wobei der Gasstrom Stickstoff, Luft, Kohlendioxid oder Mischungen davon umfasst;
(iii) wobei der Gasstrom aus heißem Stickstoff besteht.

15. Verfahren zur Bildgebung eines Magen-Darm-Trakts bei einem Patienten, umfassend ein Verabreichen einer oralen Formulierung nach einem der Ansprüche 1-7 an den Patienten, optional ist der Magen-Darm-Trakt der obere Magen-Darm-Trakt.

## Revendications

1. Formulation orale comprenant un agent d'imagerie iodé et au moins un agent de masquage de goût, l'agent d'imagerie iodé étant encapsulé dans l'agent de masquage de goût sous forme de particules, l'agent d'imagerie iodé étant un composé organique ou un polymère hydrosoluble contenant un ou plusieurs substituants iodés, et où l'agent de masquage de goût est choisi parmi un ou plusieurs polymères, tensioactifs et sucres.

2. Formulation orale selon la revendication 1, dans laquelle l'agent d'imagerie est l'iopamidol, l'iohexol ou l'iodixanol.

3. Formulation orale selon la revendication 1 ou 2, dans laquelle l'agent d'imagerie représente jusqu'à 90 % en poids.

4. Formulation orale selon la revendication 1, comprenant en outre l'une quelconque ou plusieurs des caractéristiques suivantes :
(i) un ou plusieurs agents de masquage de goût sont choisis parmi l'acétate succinate d'hydroxypropylméthylcellulose, le poly(acide méthacrylique-méthacrylate de méthyle) et le palmitostéarate de glycéryle ;
(ii) l'agent de masquage de goût est l'acétate succinate d'hydroxypropylméthylcellulose ;
(iii) l'agent de masquage de goût est le poly(acide méthacrylique-méthacrylate de méthyle).

5. Formulation orale selon l'une quelconque des revendications 1 à 4, comprenant en outre un ou plusieurs des excipients, dans laquelle l'excipient est choisi dans le groupe constitué de polymères, de tensioactifs, de lipides ou de cires, d'acides gras, de sucres et d'aromatisants.

6. Formulation orale selon l'une quelconque des revendications 1 à 5, dans laquelle les particules ont au moins l'une des caractéristiques suivantes :
(i) une taille de particules comprise entre 1 et 500 µm de diamètre ;
(ii) une surface comprise entre 0,5 et 100 m²/g mesurée à l'aide de la méthode de surface spécifique de Brunauer-Emmett-Teller (BET) ;
(iii) une cohésion entre particules et une résistance bioadhésive maximale allant jusqu'à 500 kPa, mesurée à l'aide d'un rhéomètre pour poudre.

7. Formulation orale selon l'une quelconque des revendications 1 à 6, dans laquelle la formulation est iso-osmotique ou hypo-osmotique.

8. Procédé de préparation d'une formulation selon l'une quelconque des revendications 1 à 7, le procédé comprenant :
(a) l'introduction d'un mélange fondu comprenant un ou plusieurs agents d'imagerie iodés et un ou plusieurs des agents de masquage de goût dans une buse ;
(b) l'atomisation dudit mélange fondu en gouttelettes ; et
(c) le refroidissement desdites gouttelettes en particules.

9. Procédé selon la revendication 8, comprenant en outre la fusion d'un ou de plusieurs agents d'imagerie iodés comprenant facultativement un ou plusieurs des excipients avant l'étape (a), afin d'obtenir le mélange fondu ; dans lequel, éventuellement, la fusion est effectuée à une température entre 20 et 250 °C.

10. Procédé selon la revendication 8 ou 9, dans lequel le refroidissement est favorisé par un flux de gaz ou de liquide, facultativement, dans lequel le flux de gaz ou de liquide est l'un des suivants :
(i) ledit flux de gaz ou de liquide est à une température initiale entre -40 et 100 °C ;
(ii) ledit flux de gaz ou de liquide est à co-courant ou à contre-courant par rapport à la direction de pulvérisation de matière fondue ;
(iii) ledit flux de gaz ou de liquide est un flux de gaz comprenant de l'azote, de l'air, du dioxyde de carbone ou leurs mélanges ;
(iv) ledit flux de gaz ou de liquide est un flux de gaz combiné avec un flux de liquide comprenant du dioxyde de carbone liquide ou de l'azote liquide.

11. Procédé de préparation d'une formulation selon l'une quelconque des revendications 1 à 7, le procédé comprenant :
(a) le mélange d'un ou de plusieurs des agents iodés et d'un ou de plusieurs agents de masquage de goût dans un solvant ou un mélange de solvants pour obtenir une solution ou une suspension ;
(b) l'introduction et l'atomisation de ladite solution ou suspension dans une chambre de séchage pour obtenir des gouttelettes ; facultativement, l'introduction est effectuée dans une buse et
(c) le séchage desdites gouttelettes en particules.

12. Procédé selon la revendication 11, dans lequel le séchage est favorisé par un flux de gaz ou de liquide, facultativement, le séchage est effectué à une température entre 20 et 300 °C.

13. Procédé selon l'une quelconque des revendications 8, 9, 11 ou 12, dans lequel la configuration de la buse est rotative, sous pression, fluide ou ultrasonique.

14. Procédé selon la revendication 12, comprenant en outre une ou plusieurs des caractéristiques suivantes :
(i) le flux de gaz ou de liquide est à co-courant par rapport à la direction de pulvérisation de la matière fondue ;
(ii) le flux de gaz comprend de l'azote, de l'air, du dioxyde de carbone ou leurs mélanges ;
(iii) où le flux de gaz est de l'azote chaud.

15. Procédé d'imagerie du tractus gastro-intestinal chez un patient, comprenant l'administration au patient d'une formulation orale selon l'une quelconque des revendications 1 à 7, facultativement, le tractus gastro-intestinal est le tractus gastro-intestinal supérieur.
